# EUROPEAN PATENT SPECIFICATION

(11) **EP 3 140 428 B1**
(45) Date of publication and mention of the grant of the patent: **28.07.2021**
(21) Application number: 15789313.2
(22) Date of filing: 08.05.2015
(51) Int. Cl.: C12Q 1/6806, C12Q 1/6855

(54) **METHOD OF TAGGING NUCLEIC ACID SEQUENCES**
VERFAHREN ZUR MARKIERUNG VON NUKLEINSÄURESEQUENZEN
MÉTHODE D'ÉTIQUETAGE DE SÉQUENCES D'ACIDES NUCLÉIQUES

(30) Priority: 08.05.2014 US 201461990598 P; 13.11.2014 US 201462079495 P
(43) Date of publication of application: 15.03.2017
(73) Proprietor: Fluidigm Corporation, South San Francisco, CA 94080 (US)
(72) Inventor: WEST, Jason A. A., South San Francisco California 94080 (US); FOWLER, Brian, South San Francisco California 94080 (US); CHARN, Tze-Howe, South San Francisco California 94080 (US); JOHNSON, Christian F., South San Francisco California 94080 (US); UNGER, Marc A., South San Francisco California 94080 (US)
(74) Representative: Murgitroyd & Company
(86) International application number: PCT/US2015/029986
(87) International publication number: WO 2015/172080

(56) References cited:
- WO-A1-02/18629
- WO-A1-98/15652
- WO-A1-99/02727
- WO-A1-99/02727
- WO-A1-2013/131962
- WO-A2-02/46449
- WO-A2-2012/162267
- US-A1- 2004 029 113
- US-A1- 2005 100 900
- US-A1- 2009 186 349
- US-A1- 2010 035 249
- US-A1- 2011 059 556
- US-A1- 2013 203 607
- SCHOONBROODT SONIA ET AL: "Oligonucleotide-assisted cleavage and ligation: a novel directional DNA cloning technology to capture cDNAs. Application in the construction of a human immune antibody phage-display library", NUCLEIC ACIDS RESEARCH, OXFORD UNIVERSITY PRESS, GB, vol. 33, no. 9, 1 January 2005 (2005-01-01), pages E81-1, XP002574143, ISSN: 1362-4962, DOI: 10.1093/NAR/GNI080 [retrieved on 2005-05-19]
- JIAN-BING FAN ET AL: "Highly Parallel Genome-Wide Expression Analysis of Single Mammalian Cells", PLOS ONE, vol. 7, no. 2, 8 February 2012 (2012-02-08), page e30794, XP055189434, DOI: 10.1371/journal.pone.0030794
- MARDIS ELAINE R: "Next-generation DNA sequencing methods", ANNUAL REVIEW OF GENOMICS AND HUMAN GENETICS, ANNUAL REVIEWS, US, vol. 9, 1 January 2008 (2008-01-01), pages 387-402, XP002512993, ISSN: 1527-8204, DOI: 10.1146/ANNUREV.GENOM.9.081307.164359 [retrieved on 2008-06-24]
- SCHLAGER, J. J. ET AL.: 'A degenerative primer set for PCR amplification of murine immunoglobulin heavy chain' ABSTRACT, MEDICAL DEFENSE BIOSCIENCE REVIEW, PROCEEDINGS, BALTIMORE vol. 3, 12 May 1996, VIRGINIA, USA, pages 1582 - 1589, XP008185326

## Description

### FIELD OF THE INVENTION

The invention relates to nucleic acid assays and finds application in the fields of genetics, medicine and agriculture. The methods provided herein are useful for nucleic acid sequencing and gene expression analysis in heterogeneous cell populations.

### BACKGROUND

Nucleic acid sequencing is the process of determining the nucleotide order of a given nucleic acid fragment. The original chain termination method of sequencing uses sequence-specific termination of a DNA synthesis reaction using modified nucleotide substrates. New sequencing technologies are being develooped to increase the speed and reduce the cost of determining the sequence of nucleic acid in a biological sample, such as a genome or an expression library. Such methods can be appuied commercially, for example, to identify, diagnose, and potentially develop treatments for genetic or contageous diseases.

WO 99/02727 discusses a method to characterise one or more nucleic acids wherein the method comprises immobilising double stranded nucleic acids on a solid phase support, cleaving the immobilised nucleic acids with an endonuclease such that each cleaved nucleic acid has a double-stranded portion, denaturing the cleaved nucleic acids to form single-stranded cleaved nucleic acid, each oligonucleotide sequence comprising a pre-determined recognition sequence situated such that it recognises a sequence which was part of the double-stranded portion of the nucleic acid and a label specific to the recognition sequence, extending correctly hybridised olionucleotide sequences along the single-stranaded portion of the immobilised nucleic acid to form an extended strand, denaturing the extended strand from the immobilised strand and characterising the immobilised nucleic acid by identifying the size of the extended strand and identity of the recognition sequence.
US2004/0029113 discusses a method to construct libraries of genetic packages that display a member of a diverse family of peptides, polypeptides or proteins, and collectively display at least a portion of the diversity of the family. Further this document discusses a method for cleaving single-stranded nucleic acid sequences at a desired location and a method of capturing DNA molecules that comprise a member of a diverse family of DNA's and collectively comprise at least a portion of the diversity of the family.
WO2013/131962 discusses a method of tagging nucleic acid duplexes comprising the steps of:
(a) providing a transposase and transposon composition;
(b) providing one or more nucleic acid duplexes immobilised on a support; and
(c) contacting the transposase and transposon composition with the one or more nucleic acid duplexes under conditions wherein the one or more nucleic acid duplexes and transposon composition undergo a transposition reaction to produce one or more tagged nucleic acid duplexes, wherein the transposon composition comprises a double stranded nucleic acid molecule comprising a transferred strand and a non-transferred strand.

### SUMMARY OF THE INVENTION

This disclosure provides a method of forming tagged nucleic acid sequences as claimed in claim 1. A target polynucleotide is immobilized on a solid support; a recognition-oligonucleotide is hybridized thereto; the recognition-oligonucleotide-target polynucleotide hybrid is cleaved; and an adapter nucleic acid is ligated to the cleaved target polynucleotide, thereby forming a tagged nucleic acid sequence. Also provided is a method of forming a tagged single stranded cDNA; a method of forming a plurality of tagged heterogeneous nucleic acid sequences; a library of recognition-oligonucleotides; and methods for amplifying a cDNA sequence immobilized on a solid support. These methods can be used alone or in combination for integrated single cell sequencing, and can be adapted for use in a microfluidic apparatus or device.

A method of forming a tagged nucleic acid sequence according to this disclosure can include the steps of: (i) immobilizing a target polynucleotide on a solid support, thereby forming an immobilized target polynucleotide; (ii) hybridizing a recognition-oligonucleotide to said immobilized target polynucleotide, thereby forming a recognition-oligonucleotide-target polynucleotide hybrid; (iii) cleaving said recognition-oligonucleotide-target polynucleotide hybrid with a cleaving agent, thereby forming a cleaved recognition-oligonucleotide-cleaved target polynucleotide hybrid comprising a cleaved target polynucleotide; and (iv) ligating an adapter nucleic acid sequence to said cleaved

Also provided is a method of forming a plurality of tagged heterogeneous polynucleotides. This can include the steps of: (i) immobilizing a plurality of heterogeneous target polynucleotides on a solid support, thereby forming a plurality of immobilized heterogeneous target polynucleotides; (ii) hybridizing a plurality of heterogeneous recognition-oligonucleotides to said immobilized heterogeneous target polynucleotides, thereby forming a plurality of recognition-oligonucleotide-target polynucleotide hybrids; (iii) cleaving said recognition-oligonucleotide-target polynucleotide hybrids with a cleaving agent, thereby forming a plurality of cleaved recognition-oligonucleotide-cleaved target polynucleotide hybrids; and (iv) ligating an adapter nucleic acid sequence to said plurality of cleaved target polynucleotides, thereby forming a plurality of tagged heterogeneous polynucleotides.

Also discussed herein is a method of forming a tagged single stranded cDNA. This can include the steps of: (i) immobilizing a target cDNA on a solid support, thereby forming an immobilized target cDNA; (ii) hybridizing a recognition-oligonucleotide to said immobilized target cDNA, thereby forming a recognition-oligonucleotide-cDNA hybrid; (iii) cleaving said recognition-oligonucleotide-cDNA hybrid with a cleaving agent, thereby forming a cleaved recognition-oligonucleotide-cleaved cDNA hybrid; and (iv) ligating an adapter nucleic acid to said cleaved cDNA, thereby forming a tagged single stranded cDNA.

This disclosure further provides a method of forming a tagged nucleic acid sequence. This can include: (i) immobilizing a target ribonucleic acid on a solid support, thereby forming an immobilized target ribonucleic acid (RNA); (ii) synthesizing a complementary DNA (cDNA) strand, thereby forming an RNA:cDNA hybrid; (iii) cleaving the RNA:cDNA hybrid with an RNA:cDNA cleaving agent, to generate a cleaved RNA:cDNA hybrid, wherein the cDNA comprises a ligatable end; (iv) ligating an adapter oligonucleotide to the ligatable end ; and (v) removing the ribonucleic acid sequence from said RNA:cDNA hybrid, thereby forming a tagged nucleic acid sequence.

Also provided is a method of forming a plurality of tagged heterogeneous nucleic acid sequences. This can include (i) immobilizing a plurality of heterogeneous target ribonucleic acid sequences on a solid support, thereby forming a plurality of immobilized heterogeneous target ribonucleic acid sequences; (ii) reverse transcribing said immobilized heterogeneous target ribonucleic acid sequences, thereby forming a plurality of heterogeneous RNA:DNA hybrids; (iii) cleaving said plurality of heterogeneous RNA:DNA hybrids with an RNA:DNA cleaving agent, thereby forming a plurality of cleaved RNA:DNA hybrids; (iv) ligating an adapter nucleic acid sequence to said plurality of cleaved RNA:DNA hybrids; and (v) removing said ribonucleic acid sequences from said cleaved RNA:DNA hybrids, thereby forming a plurality of tagged heterogeneous nucleic acid sequences.

Such methods can be used to prepare a library of recognition-oligonucleotides that comprise a plurality of heterogeneous recognition-oligonucleotides each comprising a restriction enzyme recognition sequence flanked by degenerate nucleic acid sequences. The cleaving agent may be a restriction enzyme, and the library may be included as part of a microfluidic device.

This invention further provides a method of amplifying a cDNA sequence as claimed in claim 8. Further disclosed is a method that includes: (i) immobilizing an RNA molecule extracted from an isolated cell on a solid support, thereby forming an immobilized ribonucleic acid sequence; (ii) reverse transcribing said immobilized ribonucleic acid sequence, thereby forming an immobilized RNA:DNA hybrid; (iii) removing said ribonucleic acid sequence from said RNA:DNA hybrid, thereby forming an immobilized cDNA sequence; (iv) hybridizing a recognition oligonucleotide to said immobilized cDNA sequence, thereby forming a recognition- oligonucleotide:DNA hybrid; (v) cleaving said recognition oligonucleotide:cDNA hybrid with a cleaving agent, thereby forming a cleaved recognition oligonucleotide:cleaved cDNA hybrid; (vi) ligating an adapter nucleic acid sequence to said cleaved cDNA, thereby forming a tagged cDNA sequence; (vii) hybridizing said tagged cDNA sequence to an amplification nucleic acid sequence under conditions allowing for PCR amplification, thereby amplifying a cDNA sequence.

Another method provided in this disclosure is a method of amplifying a cDNA sequence. This can include (i) immobilizing an RNA molecule extracted from an isolated cell on a solid support, thereby forming an immobilized ribonucleic acid sequence; (ii) reverse transcribing said immobilized ribonucleic acid sequence, thereby forming an immobilized RNA:DNA hybrid; (iii) cleaving said RNA:DNA hybrid with an RNA:DNA cleaving agent, thereby forming a cleaved RNA:DNA hybrid; (iv) ligating an adapter nucleic acid sequence to said cleaved RNA:DNA hybrid; (v) removing said ribonucleic acid from said cleaved RNA:DNA hybrid, thereby forming a tagged cDNA sequence; and (vi) contacting said tagged cDNA sequence with an amplification nucleic acid sequence under conditions allowing for PCR amplification, thereby amplifying said cDNA sequence.

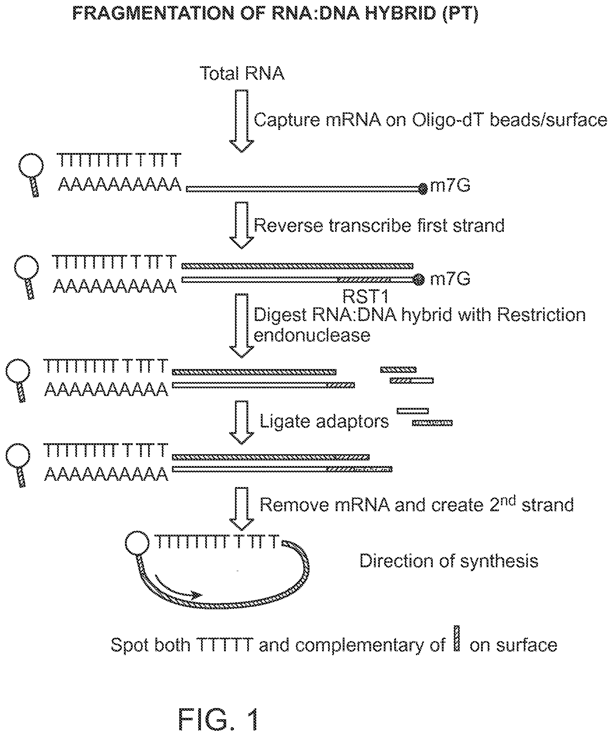

In one aspect, a method of forming a tagged nucleic acid sequence is provided. The method involves (i) immobilizing a target ribonucleic acid on a solid support, thereby forming an immobilized target ribonucleic acid (RNA); (ii) synthesizing a complementary DNA (cDNA) strand, thereby forming an RNA:cDNA hybrid; (iii) cleaving the RNA:cDNA hybrid with an RNA:cDNA cleaving agent, to generate a cleaved RNA:cDNA hybrid, wherein the cDNA comprises a ligatable end; (iv) ligating an adapter oligonucleotide to the ligatable end ; and (v) removing the ribonucleic acid sequence from said RNA:cDNA hybrid, thereby forming a tagged nucleic acid sequence. An embodiment of this approach is illustrated in Figs. 1-7.

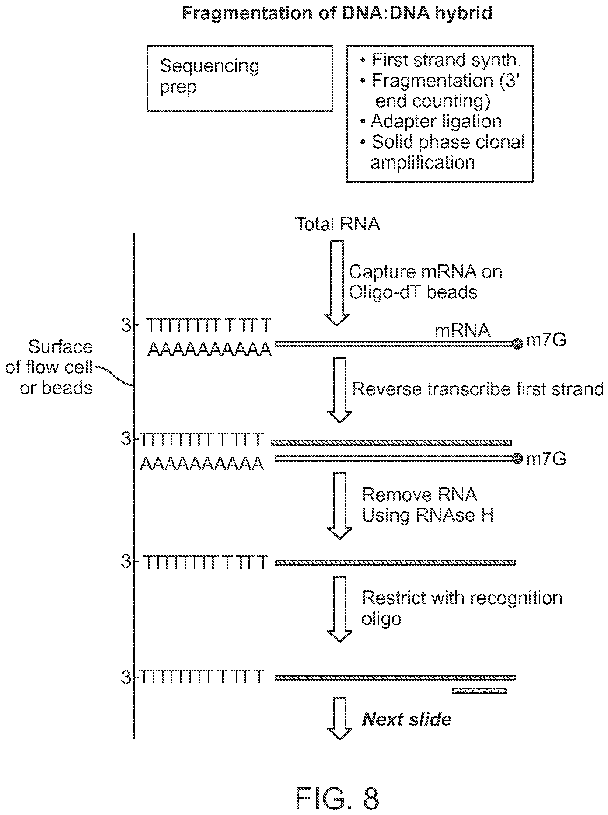

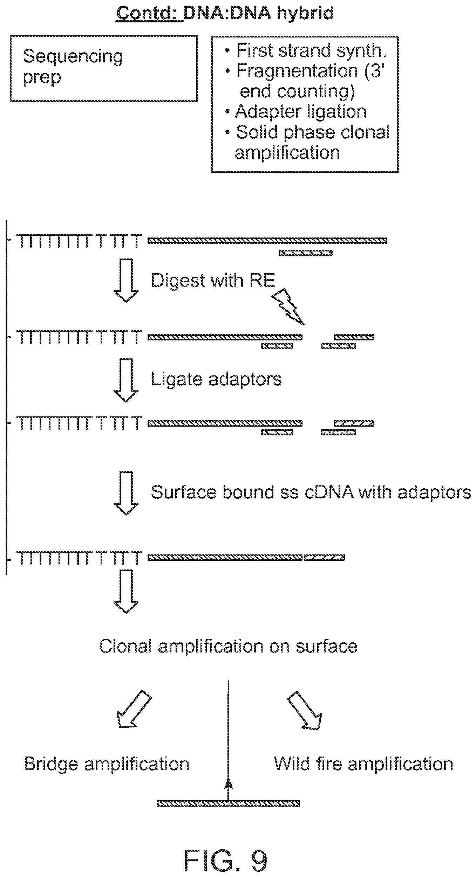

In a further aspect, a method of forming a tagged nucleic acid sequence is provided. The method involves(i) immobilizing a target ribonucleic acid on a solid support, thereby forming an immobilized target ribonucleic acid (RNA); (ii) synthesizing a complementary DNA (cDNA) strand and removing the target RNA;,(iii) hybridizing a recognition-oligonucleotide to the immobilized target cDNA, thereby forming a recognition-oligonucleotide:cDNA hybrid; (iii) cleaving the recognition-oligonucleotide:cDNA hybrid with a cleaving agent, thereby forming a cleaved recognition-oligonucleotide:cleaved dDNA hybrid, wherein the cDNA comprises a ligatable end; and (iv) ligating an adapter oligonucleotide to the ligatable end, thereby forming a tagged nucleic acid sequence. An embodiment of this approach is illustrated in Figs. 8-9.

### BRIEF DESCRIPTION OF THE DRAWINGS

**Fig. 1** is a stepwise depiction of a method for tagging immobilized nucleic acid sequences.
**Fig. 2** illustrates an RNA annealed to an anchor polynucleotide. **Fig. 3** illustrates a first strand cDNA and RNA:cDNA hybrid. **Fig. 4** illustrates the result of cleaving an RNA:cDNA hybrid using a restriction endonuclease. **Fig. 5** illustrates an adaptor oligonucleotide ligated to the free 3' end of the cleaved cDNA molecule.
**Fig. 6A** illustrates a related approach in which the single-stranded overhang of RNA:cDNA hybrid is contributed by the cDNA molecule. **Fig. 6B** illustrates another approach in which the single-stranded overhang of RNA:cDNA hybrid is contributed by the RNA molecule. **Fig. 7** illustrates the result of removing the RNA from the RNA:cDNA hybrid.
**Fig. 8** is a stepwise depiction of another method for tagging immobilized nucleic acid sequences in which a cDNA:Oligonucleotide hybrid is cleaved. **Fig. 9** is a stepwise depiction of another method for tagging immobilized nucleic acid sequences in which a cDNA is amplified on a surface.
**Figs. 10****,** **11****,** **12****, and** **13** illustrate different steps in the synthesis of the cDNA second strand by bridge amplification. **Fig. 14** shows how one of the strands may be removed before sequencing, which results in a lawn of single strands to be sequenced. **Figs. 15** **and** **16** illustrate so-called wildfire amplification in which polyadenylated mRNA is prepared by hybridizing to an immobilized poly(T) sequence. **Fig. 17** illustrates on-chip sequencing using a bead-based sequencing reaction.
**Fig. 18** is a fluorscence image showing that signals from individual beads can be distinguished when sequencing beads (or a surrogate) are seperated by filler beads. **Fig. 19** is another fluorwescence image showning how to distinguish individual sequencing beads in the absense of filler beads.
**Fig. 20** is a depiction in which mRNA and beads are combined before entry into the first chamber, following which amplification and sequence imaging re carried out In a second chamber.
**Fig. 21** is a chart showing possible chemistry for attachment of nucleic acids to a surface. **Fig. 22** is a stepwise depiction of the conjugation of an oligonucleotide onto a glass surface.
**Fig. 23** is an example of an apparatus in which signal from the second chamber is collected using a camera.

### DETAILED DESCRIPTION OF THE INVENTION

Provided herein are methods and compositions for tagging and amplifying nucleic acid sequences. The methods and compositions provided are useful for, *inter alia,* single cell sequencing procedures and may be used to determine RNA expression profiles of individual cells of a heterogeneous cell population.
**Part 1** describes methods for tagging immobilized nucleic acid sequences.
**Part 2** describes amplification and sequencing tagged nucleic acid sequences.
**Part 3** methods for sequencing and data collection.
**Part 4** describes integrated microfluidic devices.
**Part 5** describes additional description about certain elements described in Parts 1-4.

### PART 1: Methods for Tagging Immobilized Nucleic Acid Sequences

In one aspect the disclosure relates to immobilizing a target RNA, producing a cDNA sequence complementary to at least a portion of the target RNA, cleaving the cDNA sequence to produce a new free terminus, and tagging the cDNA by ligating an adaptor sequence to the new free terminus.

### 1. First Approach - In which a cDNA:RNA hybrid is cleaved

### 1.1. Produce cDNA

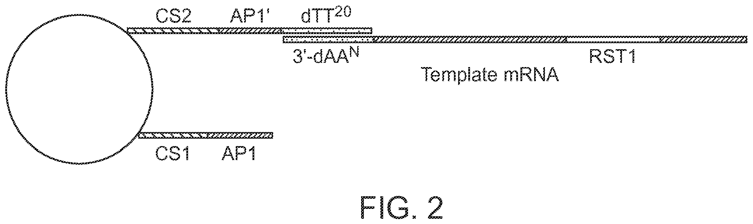

A first approach is summarized in **FIG. 1** and illustrated in **FIGS. 2 to 6B****.** It will be appreciated that **FIGS. 1-6** show an exemplary embodiment of the disclosure which is not part of the claimed invention. In the illustrated embodiment, an RNA **200,** typically a mRNA, is immobilized (or "captured") on a surface **100. See** **FIG. 2****.** In some embodiments surface **100** is a bead. In other embodiments the surface may be substantially planar. As illustrated, the RNA may be captured by annealing to anchor polynucleotide **50** which is immobilized on the surface. In one approach a polyA tail **55** of the mRNA anneals to an oligo d(T) portion **51** of the anchor polynucleotide. Oligo d(T) portion **51** is suitable for priming a polymerase (e.g., reverse transcriptase) reaction, e.g., and comprises a free 5' end (**FIG. 2**). In some embodiments the RNA is annealed to a sequence of the anchor polynucleotide other than oligo d(T), such as a transcript specific sequence. In some embodiments the RNA does not comprise a poly(A) tail. For simplicity, the RNA will be referred to as mRNA and the capture sequence to which the RNA anneals will be referred to as an oligo d(T) capture sequence.

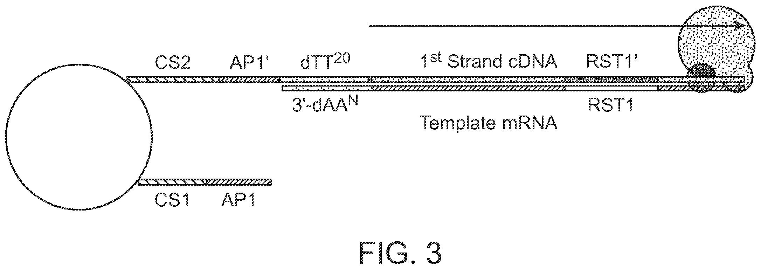

As discussed in greater detail below, in some embodiments the anchor polynucleotide comprises, in addition to oligo d(T) capture sequence, an amplification primer sequence (AP1') **53.** The anchor polynucleotide may also comprise a cut site sequence (CS2) **52.** The cut site sequence may be a restriction endonuclease recogntion sequence
The immobilized mRNA is reverse transcribed from the oligo d(T) primer, producing 1^{st} strand cDNA **301** and RNA:cDNA hybrid **300 (****FIG. 3****)** immobilized on the surface. Following the polymerization reaction, optionally the reverse transcriptase may be inactivated, e.g., by heating.

### 1.2. Cleave RNA:cDNA hybrid

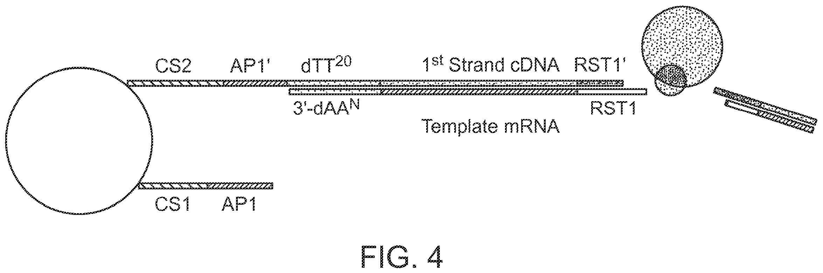

The RNA:cDNA hybrid is cleaved using a restriction endonuclease (RE) to produce a free RNA 5' terminus and a free DNA 3' terminus (**FIG. 4****).** The RE cleaves at a restriction site (RST1:RST1') in the cDNA:RNA hybrid. In one embodiment the termini resulting from the cleavage are staggered creating a sticky end **(****FIG. 4****).** In one embodiment the RE creates a blunt end. Features of the RE are discussed below. After the cleavage step the RE may be inactivated (e.g., heat inactivated).

### 1.3. Ligate Adaptor Oligonucleotide

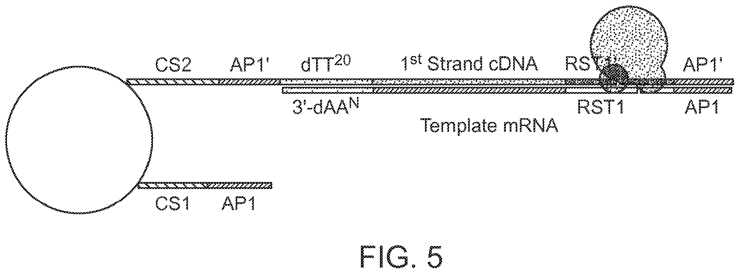

An adaptor oligonucleotide **310** is ligated to the free 3' end of the cleaved cDNA molecule, e.g., using a DNA ligase. Any suitable method of ligation may be used. As illustrated in **FIG. 5****,** the adaptor oligonucleotide comprises a second copy of amplification primer sequence (AP1') **53.**

In one approach, ligation of the adaptor oligonucleotide comprises annealing a partially double stranded polynucleotide **320** (one strand of which is the adaptor oligonucleotide) to a sticky end created by the RE cleavage. The nature of the sticky end will depend, typically, on the choice of RE. In one approach, a single-stranded overhang of cleaved RNA:cDNA hybrid is contributed by the template mRNA molecule, as illustrated in **FIG. 4****.** In this approach, a partially double-stranded adaptor construct, in which a protruding strand is complementary to the single-stranded overhang of the RNA molecule, is annealed to the RNA. The 5' end of the protruding strand of the adaptor is ligated to the 3' end of the cleaved cDNA, thereby producing a tagged nucleic acid molecule.

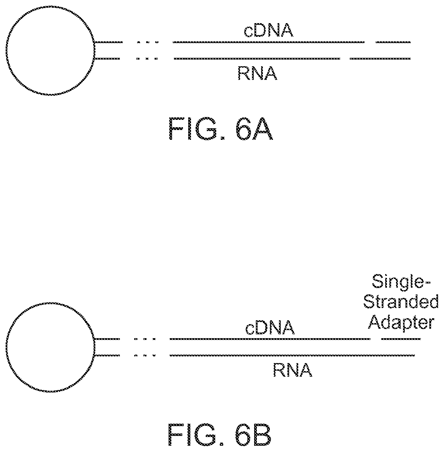

In a related approach, the single-stranded overhang of RNA:cDNA hybrid is contributed by the cDNA molecule, as illustrated in **FIG. 6A**. In this approach, a partially double-stranded adaptor construct, in which a protruding strand has is complementary to the single-stranded overhang of the cDNA molecule, is annealed to the cDNA. The 5' end of the non-protruding strand of the adaptor is ligated to the 3' end of the cleaved cDNA, thereby producing a tagged nucleic acid molecule.

In another approach in which the single-stranded overhang of RNA:cDNA hybrid is contributed by the RNA molecule, a single-stranded adaptor oligonucleotide a 5' region complementary to the single-stranded overhang of the RNA molecule (rather than a partially double-stranded molecule) is annealed RNA, with, as illustrated in **Fig. 6B**. The 5' end of the single-stranded adaptor oligonucleotide is ligated to the 3' end of the cleaved cDNA, thereby producing a tagged nucleic acid molecule.

In another embodiment, the adaptor does not anneals directly adjacent to the cDNA, and a polymerase is used for gap filling prior toligation.

In another approach, cleavage of the cDNA:RNA hybrid creates a blunt end. Ligation of the adaptor oligonucleotide can be accomplished by ligating a double stranded polynucleotide comprising adaptor oligonucleotide **310** to the blunt end. This results in a heterogeneous mixture of identically tagged cDNA molecules.

An exemplary ligase for ligation of an oligonucleotide to a single stranded cDNA is T4 RNA ligase 1 (Troutt et al., 1992, Proc. Natl, Acad. Sci. USA. 89:9823-25), optionally in the presence of hexamine cobalt chloride.

After the ligation step the ligase may be inactivated (e.g., heat inactivated).

### 1.4. Remove RNA

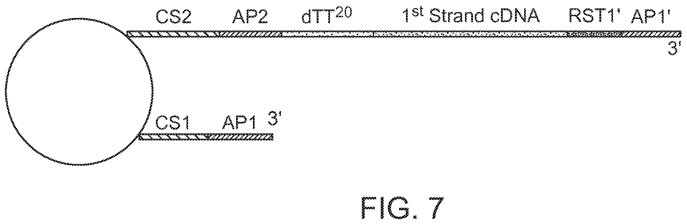

The RNA is removed from the RNA:cDNA hybrid. The RNA may be removed enzymatically, chemically, or thermally. In some embodiments the RNA is degraded. The result is an immobilized bound single-stranded cDNA tagged with an adaptor **(****FIG. 7****),** *i.e.,* a tagged nucleic acid molecule. In one embodiment RNA is removed from the hybrid using a ribonuclease, such as RNase H.

### 1.5. Multiple Adaptor Oligonucleotides

Often, as illustrated in the drawings, a single adaptor oligonucleotide **310** is used. However, in some embodiments, two or more different adaptor oligonucleotides are used. In one approach, the different adaptor oligonucleotides are compatible with different RE sites, allowing RNAs with different RE sites to be processed in the same reaction.

In a different example, different adaptor oligonucleotides are distinguished by having different AP1' sequences. For example, a first cDNA anchor oligonucleotide **50** comprising a first AP1' sequence **53**, a first sequence-specific capture sequence, a first restriction site, and an adaptor oligonucleotide comprising the first AP1' sequence may be used in combination with a second anchor oligonucleotide **50** comprising a second AP1' sequence **53**, a second-sequence specific capture sequence different from the first, a second restriction site, and an adaptor oligonucleotide comprising the second AP1' sequence. In this fashion it is possible using multiple sequence specific capture sequences, to produce a heterogeneous mixture in which some nucleic acid species (e.g., cDNA) are tagged with one tag and some nucleic acid species (e.g., cDNA) are tagged with a different tag.

### 1.6. Additional Processing Steps

Typically, the immobilized tagged nucleic acid molecule is subjected to additional processing steps, such as clonal amplification on the surface, and sequencing, as discussed below.

### 2. Second Approach - In which a cDNA:Oligonucleotide hybrid is cleaved

A second tagging approach is illustrated in **FIGS. 8-9**. It will be appreciated that **FIGS. 8-9** show a certain embodiment, to which the invention is not limited, the invention being limited by the appended claims.

### 2.1. Produce cDNA

In this embodiment, an mRNA **60** is immobilized on a surface (e.g., bead) **61** as described in **§1.1**, above, e.g., via annealing of a polyA tail to an immobilized oligo d(T)-containing anchor polynucleotide. The immobilized mRNA is reverse transcribed, as also described in **§1.1,** above, to produce an RNA:cDNA hybrid **62** in which the first strand cDNA **63** is immobilized on the surface.

### 2.2. Remove RNA

The RNA is removed from the hybrid, e.g., using chemical, thermal or enzymatic methods, such as treatment with a ribonuclease such as RNase H, leaving the single-stranded immobilized cDNA.

### 2.3. Produce cDNA: Recognition Oligonucleotide Hybrid

The immobilized cDNA is subsequently hybridized to a recognition oligonucleotide **64**, which is at least partially complementary to a portion of the cDNA, rendering a portion of the cDNA double-stranded and susceptible to digestion with a restriction endonuclease. The degree of complementarity between the cDNA and the recognition oligonucleotide is a degree sufficient to result in a double-stranded region (e.g., the cDNA:oligo hybrid) that can be recognized by a specified restriction endonuclease. which recognizes the oligonucleotide:cDNA hybrid and cleaves the cDNA. Typically the recognition oligonucleotide will be at least 12, usually at least 15 and sometimes at least 25 bases in length.

In some embodiments, an assay is carried out using a single recognition oligonucleotide. In some embodiments multiple different recognition oligonucleotides are used. When different recognition oligonucleotides are used, they may anneal to different cDNA sequences to create double-stranded regions recognized by different restriction endonucleases. Alternatively, they may anneal to different cDNA sequences to create double-stranded regions recognized by the same restriction endonuclease, but having flanking sequences (for example) that increase the stability of the oligo:cDNA hybrid. This provides increased flexibility when working with a populations of highly heterogeneous sequences, because a combination of different restriction endonucleases to generate a ligatable end, or a single restriction endonuclease may be used to generate ligatable ends from substrates with diverse restriction site or flanking sequences.

In some cases a degenerate population of recognition oligonucleotides, as described in **Section 4.2,** is used to generate ligatable ends of a heterogeneous population.

### 2.4. Cleave cDNA:Oligonucleotide hybrid

The oligonucleotide:cDNA hybrid comprising a restriction endonuclease recognition site is recognized by a specified restriction endonuclease (or endonucleases) cleaves the immobilized cDNA. The action of the RE produces a free 3' cDNA terminus. Depending on the choice of recognition oligonucleotide(s) and restriction endonuclease(s), the immobilized cleavage product can have a blunt end or sticky end. The sticky end can comprise a single-stranded overhang contributed by the cDNA or by the recognition oligonucleotide, analogous to the cDNA:RNA cleavage products discussed above in **§1.3**, above, for RNA:cDNA hybrids.

### 2.5. Ligate Adaptor Oligonucleotide

An adaptor oligonucleotide may be ligated to the free 3' end of the cleaved cDNA molecule, analogous to the description in **§1.3** above for RNA:cDNA hybrids, resulting in a tagged cDNA or, more generally, a surface or plurality of surfaces comprising a heterogeneous population of tagged immobilized cDNAs.

### 2.6. Additional Processing Steps

Typically, the immobilized tagged nucleic acid molecule is subjected to additional processing steps, such as clonal amplification on the surface, and sequencing, as discussed below.

### 3. Selection Of A Restriction Endonuclease(s)/Restriction Endonuclease Recognition Site(s)

### 3.1. General Properties

As discussed above, prior to addition of the Adaptor Oligonucleotide, the cDNA:RNA hybrid or the cDNA:Recognition Oligonucleotide hybrid is cleaved with a restriction endonuclease. As used herein, restriction endonucleases are enzymes that cleave DNA at or near specific recognition nucleotide sequences (restriction sites). See, e.g., Roberts et al., 2007 "REBASE-enzymes and genes for DNA restriction and modification," Nucleic Acids Res 35 (Database issue): D269-70; see http site rebase.neb.com). For illustration and not limitation, restriction enzymes for use in the present invention include Type I enzymes (EC 3.1.21.3), Type II enzymes (EC 3.1.21.4), e.g., Type IIs and Type IIP, and Type III enzymes (EC 3.1.21.5). Restriction enzymes occur in nature, may be recombinantly produces, and may be artificial (e.g., comprising sequences from multiple different proteins).

In some embodiments, the RE produces a 3' protruding sticky end. In some embodiments, the RE produces a 5' protruding sticky end. In some embodiments, the RE produces a blunt end.

In some embodiments the RE cleaves DNA and RNA strands of a RNA:DNA hybrid.

In some embodiments,the RE is BaeG1, which recognizes the following restriction site:

5'...GKGOMC...3'
3'...CMCGKG...5'

In some embodiments,the RE is a Type-IIs restriction endonuclease that cleaves 2 to 30 nucleotides away from the recognition site. Some Type-IIs endonucleases are "exact cutters" that cut a known number of bases away from their recognition sites. In some embodiments, the overhang of the sticky end is at least 2 bases in length, at least least 2 bases in length, least 3 bases in length, at least 4 bases in length, at least 5 bases in length, at least 6 bases in length, or at least more than 6 bases in length.

The selection of the restriction endonuclease or restriction endonucleases, and, in the case of the cDNA:Recognition Oligonucleotide hybrid, the design of the Recognition Oligonucleotide sequence takes into account several desired goals.
i) For the *First Approach* - *In which a cDNA:RNA hybrid is cleaved,* the enzyme should be capable of cleaving such a hybrid.
ii) The site(s) should be present in a large number of different RNA species, so that a sufficient number of cDNAs is tagged. A "sufficient number" may be most, almost all, a majority, or a subset less than a majority.
iii) The length of the immobilized cleaved cDNA should be sufficient for the sequencing goal (usually at least 15-20 bases) and sufficiently far away from the substrate on which it is immobilized for sequencing reactions to occur. As discussed below, only a portion of the cDNA or genomic sequence is needed to identify many RNA or genomic DNA sequences (e.g., partial sequence is sufficient to identify a specific RNA by reference to a databse of known sequences.
iv) The length of the immobilized cleaved cDNAs should be compatible with the amplification method used.

### 3.2. Cleavage of DNA:RNA hybrids

For method in which a cDNA:RNA hybrid is cleaved, suitable enzymes will recognize such hybrids. For example and without limitation, suitable enzymes include *Ava*II, *Avr*II*, Ban*I, *Hae*III, *Hinf*I *and Taq*I (see Murray et al., 2010, Sequence-specific cleavage of RNA by Type II restriction enzymes" Nucleic Acids Res. 38:8257-68).

### 3.3. Cleavage Frequency

In one approach the restriction enzyme site(s) occurs in the RNA (cDNA) of the source organism at a frequency that allows for the formation of target polynucleotides with an average length of about 250 base pairs, e.g., 50-500 basepairs, or 150-350 basepairs. Preferably, most (e.g., more than 50%, more than 75%, more than 80%, more than 90%, or more than 95%) of the immobilized cDNAs are cleaved and tagged, and of the immobilized tagged cDNAs most (e.g., more than 50%, more than 75%, more than 80%, more than 90%, or more than 95%) have a length of at least 25 bases, or at least 40 bases, or at least 50 bases, or at least 75 bases, or at least 100 bases, or at least 150 bases.

**Table 1**, below, provides the specificities for a selection of REs and provides the calculated average fragment length based on human genomic DNA (adapted from of New England BioLabs; www.neb.com/tools-and-resources/selection-charts/frequencies-of-restriction-sites). Most RNA samples can be expected to deviate from the frequency and lenghts calculated for genomic sequences, but Table 1 illustrates that enzymes (individually or in combination) can be selected to achieve goals (i)-(iii) above. It should be clearly understood that not all of the enzymes in **Table 1** will be useful (e.g., the *Bst*EII recognition site may be too infrequent for most samples) and not all of the useful enzymes are included in Table 1 (e.g., *Bae*G1 is not in Table 1).

Alternatively, enzyme(s) can be selected based on emperical analysis of the lengths of cDNAs produced by digestion with the enzyme.

**TABLE 1**

| Cleaving Agents And Frequencies Of Restriction sites In The Human Genome | | | | |
|---|---|---|---|---|
| Enzyme | Specificity | Site Counts | Sites Per Megabase | Average Fragment Length (bp) |
| BstEII | GGTNACC | 335865 | 114 | 8746 |
| BamHI | GGATCC | 365999 | 124 | 8026 |
| SmaI | CCCGGG | 376939 | 128 | 7793 |
| XmaI | CCCGGG | 376939 | 128 | 7793 |
| SapI | GCTCTTC | 377161 | 128 | 7789 |
| SpeI | ACTAGT | 400286 | 136 | 7339 |
| EcoRV | GATATC | 446473 | 151 | 6580 |
| ApaI | GGGCCC | 460339 | 156 | 6381 |
| ApaLI | GTGCAC | 496255 | 168 | 5920 |
| ScaI | AGTACT | 543793 | 185 | 5402 |
| SphI | GCATGC | 550951 | 187 | 5332 |
| MfeI | CAATTG | 564303 | 192 | 5206 |
| EciI | GGCGGA | 571273 | 194 | 5142 |
| HgaI | GACGC | 571889 | 194 | 5137 |
| AvrII | CCTAGG | 594956 | 202 | 4938 |
| BciVI | GTATCC | 696093 | 236 | 4220 |
| BceAI | ACGGC | 705176 | 240 | 4166 |
| BsaAI | YACGTR | 713800 | 242 | 4115 |
| FnuDII | CGCG | 733938 | 249 | 4003 |
| BcII | TGATCA | 738785 | 251 | 3976 |
| NcoI | CCATGG | 759594 | 258 | 3867 |
| BgIII | AGATCT | 774732 | 263 | 3792 |
| EcoRI | GAATTC | 847341 | 288 | 3467 |
| XbaI | TCTAGA | 850998 | 289 | 3452 |
| HindIII | AAGCTT | 860361 | 292 | 3414 |
| NdeI | CATATG | 903360 | 307 | 3252 |
| StuI | AGGCCT | 925728 | 315 | 3173 |
| AvaIII | ATGCAT | 933357 | 317 | 3147 |
| BspHI | TCATGA | 978289 | 333 | 3003 |
| PvuII | CAGCTG | 1084260 | 369 | 2709 |
| PpuMI | RGGWCCY | 1085138 | 369 | 2707 |
| AccI | GTMKAC | 1101063 | 374 | 2668 |
| BscGI | CCCGT | 1231061 | 419 | 2386 |
| PstI | CTGCAG | 1321469 | 449 | 2223 |
| BspMI | ACCTGC | 1438128 | 489 | 204 3 |
| FauI | CCCGC | 1439772 | 490 | 2040 |
| AfIIII | ACRYGT | 1485394 | 505 | 1978 |
| BsmI | GAATGC | 1549349 | 527 | 1896 |
| HgiCI | GGYRCC | 1550876 | 527 | 1894 |
| EsaBC3I | TCGA | 1603339 | 545 | 1832 |
| TaqI | TCGA | 1603339 | 545 | 1832 |
| PsiI | TTATAA | 1647911 | 560 | 1783 |
| BfiI | ACTGGG | 1706593 | 580 | 1721 |
| HhaI | GCGC | 1756498 | 597 | 1672 |
| HinP1I | GCGC | 1756498 | 597 | 1672 |
| HpaII | CCGG | 2317719 | 788 | 1267 |
| SspI | AATATT | 2377267 | 809 | 1236 |
| SmII | CTYRAG | 2727866 | 928 | 1077 |
| NspI | RCATGY | 3104357 | 1056 | 94 6 |
| StyI | CCWWGG | 3114107 | 1060 | 943 |
| SfeI | CTRYAG | 3531009 | 1201 | 832 |
| BscAI | GCATC | 3652924 | 1243 | 804 |
| SfaNI | GCATC | 3652924 | 1243 | 804 |
| MlyI | GAGTC | 3931690 | 1338 | 747 |
| PleI | GAGTC | 3931690 | 1338 | 747 |
| Tsp45I | GTSAC | 4021757 | 1369 | 730 |
| AciI | CCGC | 4206123 | 1431 | 698 |
| TfiI | GAWTC | 4984358 | 1696 | 589 |
| CviQI | GTAC | 5115077 | 1741 | 574 |
| PabI | GTAC | 5115077 | 1741 | 574 |
| RsaI | GTAC | 5115077 | 1741 | 574 |
| FokI | GGATG | 5201113 | 1770 | 565 |
| BbvI | GCAGC | 5290042 | 1800 | 555 |
| R1.BceSIV | GCAGC | 5290042 | 1800 | 555 |
| TseI | GCWGC | 5290042 | 1800 | 555 |
| Cac8I | GCNNGC | 5461330 | 1859 | 538 |
| BsrI | ACTGG | 5741305 | 1954 | 512 |
| HphI | GGTGA | 6007328 | 2044 | 489 |
| BccI | CCATC | 6170919 | 2100 | 476 |
| BthCI | GCNGC | 6209919 | 2113 | 4 73 |
| Fnu4HI | GCNGC | 6209919 | 2113 | 473 |
| ApoI | RAATTY | 6382371 | 2172 | 460 |
| MjaIV | GTNNAC | 6385575 | 2173 | 460 |
| BsmAI | GTCTC | 6631583 | 2257 | 443 |
| Hin4II | CCTTC | 7059911 | 2403 | 416 |
| NlaIV | GGNNCC | 7118874 | 2423 | 413 |
| BspKT6I | GATC | 7199381 | 2450 | 408 |
| BspNCI | CCAGA | 7282435 | 2479 | 403 |
| HaeIII | GGCC | 8562227 | 2914 | 343 |
| TspRI | CASTG | 8765234 | 2983 | 335 |
| HinfI | GANTC | 8916048 | 3035 | 329 |
| Hpy188I | TCNGA | 8942142 | 3043 | 329 |
| MboII | GAAGA | 9199487 | 3131 | 319 |
| BstNI | CCWGG | 9855638 | 3354 | 298 |
| ScrFI | CCNGG | 11805089 | 4018 | 249 |
| SsoII | CCNGG | 11805089 | 4018 | 249 |
| AluI | AGCT | 13027766 | 4434 | 225 |
| CviAII | CATG | 13815688 | 4702 | 213 |
| FatI | CATG | 13815688 | 4702 | 213 |
| NlaIII | CATG | 13815688 | 4702 | 213 |
| DdeI | CTNAG | 14312039 | 4871 | 205 |
| MseI | TTAA | 19214668 | 6540 | 153 |
| MnII | CCTC | 27739484 | 9442 | 106 |

| | | | | |
|---|---|---|---|---|
| **B** = C or G or T; **D** = A or G or T; **H** = A or C or T; **K** = G or T; **M** = A or C; **N** = A or C or G or T; **R** = A or G; **S** = C or G; **V** = A or C or G; **W** = A or T; **Y** = C or T. | | | | |

### 4. Design of Recognition Oligonucleotides

In the methods provided herein a Recognition Oligonucleotide is hybridized to the immobilized target polynucleotide, thereby forming a recognition-oligonucleotide-target polynucleotide hybrid. Formation of the hybrid allows cleavage by a restriction enzyme and subsequent formation of a free DNA terminus to which a double stranded "adaptor" construct is ligated.

### 4.1. Structure of Recognition Oligonucleotide

It will be appreciated that the Recognition Oligonucleotide should be designed taking into account the considerations of §3(a)(ii)-(iv), above, because the Recognition Oligonucleotide and the RE together determine what positions in the cDNA are cleaved.

The Recognition Oligonucleotide is a single stranded nucleic acid, typically single stranded DNA. The Recognition Oligonucleotide" is generally less than 300 bases in length, and more often the Recognition Oligonucleotide is from about 10 to about 90 bases in length. For example, the In embodiments, the Recognition Oligonucleotide is about 15 to about 85 bases in length. In embodiments, the Recognition Oligonucleotide has a length in the range of 35 to 65 bases; 40 to 60 bases; 15 to 55 bases; 50 to 55 bases; In embodiments, Recognition Oligonucleotide is about 12, about 15, about 18, about 20, about 22, about 25, about 26, about 28, about 30, about 35, about 40, about 45, about 50, about 55, about 60 bases, about 65 bases, about 70 bases, about 75 bases or about 80 bases in length. In embodiments, Recognition Oligonucleotide is 26 bases in length.

In some embodiments the Recognition Oligonucleotide has a sequence exactly complementary to the portion of the sequence of the cDNA to which the Recognition Oligonucleotide hybridizes. However, hybridization between the Recognition Oligonucleotide and immobilized cDNA does not require 100% complementary. Recognition Oligonucleotide and the immobilized target polynucleotide are hybridizable when there is a sufficient degree of complementarity to avoid nonspecific binding of Recognition Oligonucleotide to non-target sequences under conditions where specific binding is desired, for example under conditions that allow for site-specific restriction enzyme digestion. Typically there is exact complementarity at the RE recognition site. In some embodiments, the Recognition Oligonucleotide (i) has the structure 5'-Aₙ-Xₘ-Bₙ-3' where each n is independently an integer from 5-40, X is a RE recognition sequence, and m is an integer from 4 to 10 and (ii) hybridizes to a cDNA sequence the structure 5'-A"ₙ-X'ₘ-B"ₙ-3', wherein A and B are nucleotide sequences complementary or partially complementary to sequence A" and B" and X is exactly complementary to X'.

### 4.2 Degenerate Recognition Oligonucleotides

In some embodiments the Recognition Oligonucleotide is a library or population of oligonucleotides in which certain positions are completely degenerate (i.e., oligonucleotides with A, T, G and C are represented), partially degenerate (i.e., oligonucleotides with two or three of the bases A, T, G and C are represented), and/or represented by a 'universal' base, such as deoxyinosine) is used.

Two types of degeneracy may be considered. First, there may be degeneracy at positions in the RE recognition site, to account for REs with more than one cleavage sequence. For example, *Bae*G1 recognizes 5'-GKGCMC-3' where K = G or T and M = A or C. In the case of *Bae*G1, for illustration and not limitation, the library could contain Recognition Oligonucleotides with four different RE recognition sites: 5'-GGGCAC-3'; 5'-GGGCCC-3'; 5'-GTGCAC-3'; 5'-and GTGCCC-3'.

The second type of degeneracy is degeneracy in the sequences flanking the RE recognition sites. In one embodiment the flanking sequences are fully degenerate, so that some oligonucleotide from the library of Recognition Oligonucleotides can hybridize to any cDNA that comprises the appropriate RE recognition site.

### 4.3. Multiple Recognition Oligonucleotides

In some embodiments, a library comprises more than one cleaving agent recognition sequence, such as two, three or four different sequences. In embodiments, the different cleaving agent recognition sequences are recognized by different cleaving agents.

### 4.4 Specific Targets

In some embodiments, recognition oligonucleotides are selected to bind only one or more particular subsets of sequences. For example, recognition oligonucleotides could be selected so that only cDNAs encoding actin are tagged.

### 4.5 Sequencing Genomic or Mitochondiral DNA

It will be recognized that methods, systems and devices described here in the context of characterizing RNA can be used for DNA sequencing, with modifications that will be clear to one of skill in the art guided by this specification (e.g., genomic DNA is fragmented and individual fragments are sequenced, single-stranded DNA is made double-stranded using a DNA-dependent DNA polymerase .

### PART 2: AMPLIFICATION AND SEQUENCING TAGGED NUCLEIC ACID SEQUENCES

Additional processing steps, as shown below, may be used to sequence the tagged molecule.

### 5.1 Clonal Amplification On Surface

In certain embodiments, the tagged cDNA templates are amplified prior to sequencing to result in a clonal (bi-clonal, or oligo-clonal) population of template molecules on the surface (e.g. on an individual bead, at a particular position on a surface, etc.). Examples of clonal amplification methods include bridge amplification and wildfire amplification. However, the invention is not limited to any particular method of amplification. Further, amplification is not required. For example, single polynucleotides may be characterized.

As discussed below, individual tagged cDNA molecules may be amplified to create clusters of copies of the same molecule, an approach useful for certain sequencing methods. In one embodiment, the mRNAs are captured in physically distinct surfaces or areas on a surface (e.g., on beads, in wells, at positions on an array). In an embodiment, the mRNAs are captured so that at least some physically distinct areas capture a single mRNA (e.g., one mRNA per bead, or one mRNA per well). In an embodiments, each some physically distinct area comprises, on average, one mRNA (e.g., on average from 0.5 to 1.5 mRNA molecules per physically distinct area).

### 5.1.1. Bridge amplification

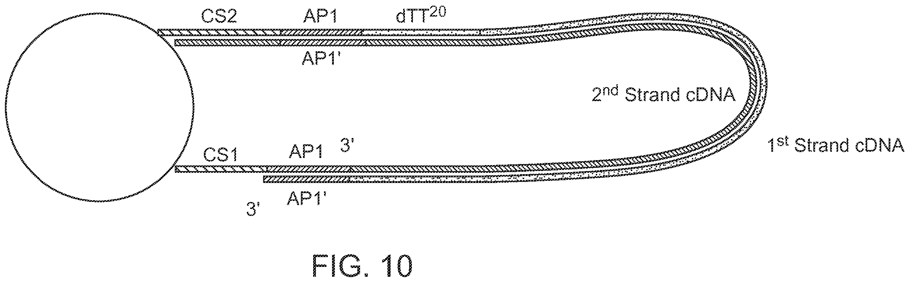

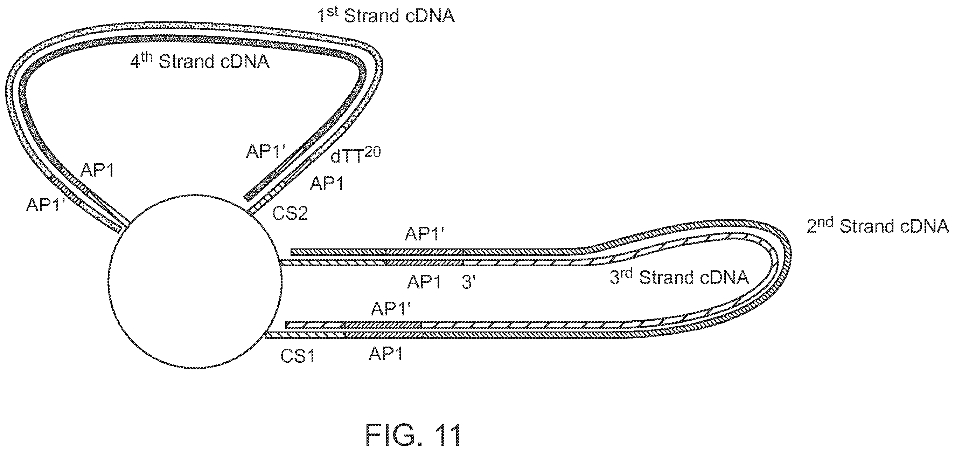

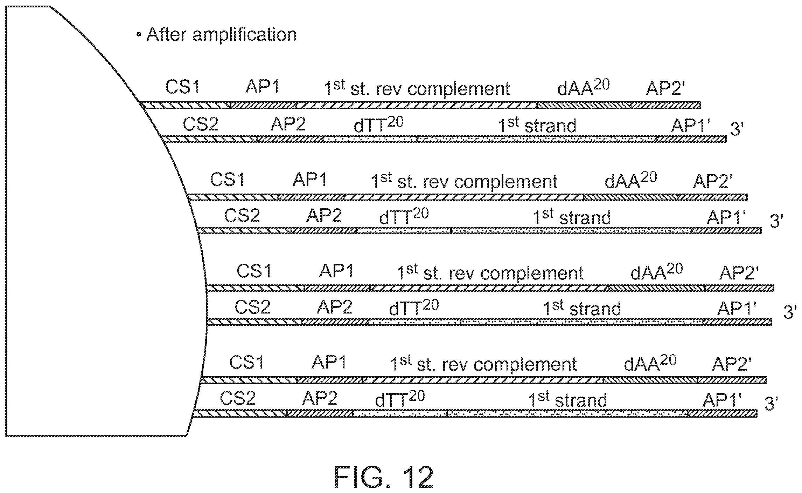

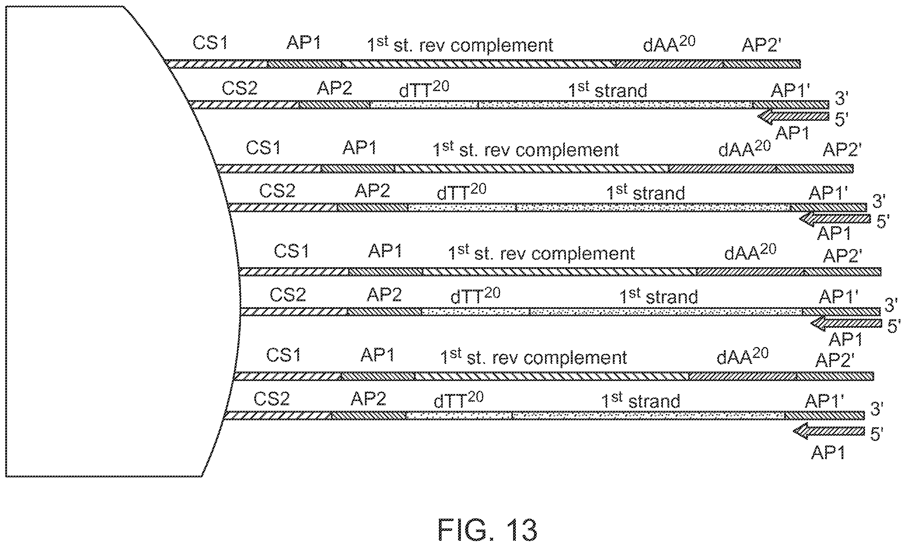

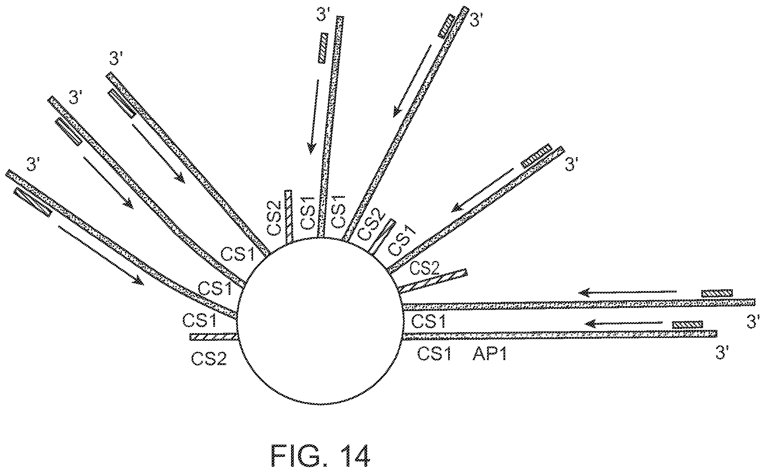

**Fig. 10** shows synthesis of the cDNA second strand. The AP1' tag sequence of the first strand hybridizes **to immobilized oligonucleotide** comprising a complementary sequence (AP1) which acts as primer to synthesize the second strand. The well-known process of bridge amplification continues as illustrated in **Figs. 11-13****.** **Fig. 12** illustrates the surface after amplification, resulting in clonal templates for sequencing. **Fig. 13** illustrates sequencing by synthesis using a primer complementary to AP1'. **Fig. 14** illustrates that one of the strands (i.e., the population of forward strands or the population of reverse strands may be removed before sequencing resulting in a lawn of single strands that may be sequenced.

As described hereinbelow, the steps above may be carried out with a large and heterogeneous mixture of mRNA molecules, such as a population of mRNA molecules from a single cell of a small number of cells.

### 5.1.2 Wildfire amplification

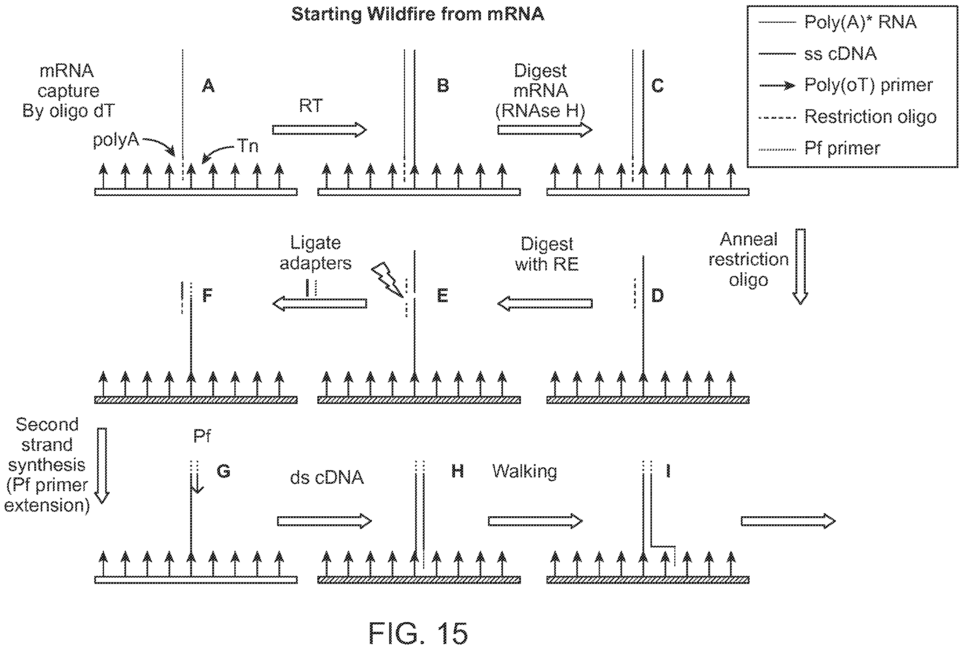

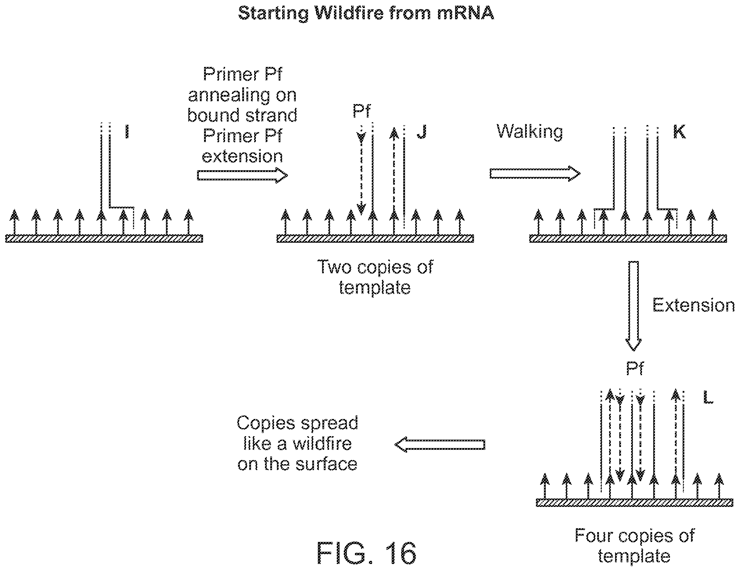

"Wildfire" amplification (Ma et al., 2013, Isothermal amplification method for next-generation sequencing" Proc Nat Acad Sci 10:14320-23)can be used for solid-phase clonal amplification. See US 2012/0156728 (Wildfire amplification) and US 2013/0203607 (WildFirePaired-End sequencing). In a modification of this approach, illustrated at **Figs. 15-16****,** polyadenylated mRNA is hybridized to an immobilized poly(T) sequence, first strand cDNA synthesis is carried out, and the RNA template is removed, leaving an immobilized first strand cDNA. The cDNA is tagged as described above, and then amplified using the Wildfire method.

### 5.2 Sequencing

Sequencing of Individual molecules (single molecule sequencing) or clonal populations can be carried out using known methods such as Solexa (Illumina) sequencing, pyrosequencing (454), SOLiD sequencing, and Polonator sequencing. See, e.g., Shendure and Ji, 2008, "Next-generation DNA sequencing" Nature Biotechnology 26:1135-45, especially Figure 3 and references cited therein. In some embodiments sequencing-by-synthesis methods are used. In some embodiments the sequencing method is a sequencing-by-synthesis method. In some embodiments, reversible terminators are used.

### 5.3 Sequencing without clonal amplification

In some embodiments, mRNA is sequenced directly, or after, or coincident with cDNA synthesis without clonal amplificaition. See, e.g., Causey et al., US Pat. Pub 20110129827 "Methods For Transcript Analysis"; Ozsolak et al., 2010 "Amplification-free digital gene expression profiling from minute cell quantities Nature Methods 7:619-21; Ozsolak et al., 2011 "Single-molecule direct RNA sequencing without cDNA synthesis" Wiley Interdiscip Rev RNA. 2011 Jul-Aug; 2(4): 565-570; Hebenstreit, 2012, "Methods, Challenges and Potentials of Single Cell RNA-seq" Biology (Basel). 1(3):658-667; Saliba et al., 2014, "Single-cell RNA-seq: advances and future challenges," Nucleic Acids Res. 42:8845-60.

### PART 3: METHODS FOR SEQUENCING AND DATA COLLECTION

### 6. Sequence Determination

Highthrouput sequencing methods are known in which a nucleic acid template to be sequenced is immobilized or positioned on a solid support, such as a bead, flow cell surface, semiconductor, or the like. A variety of different sequencing approaches may be used. For sequencing methods in which a fluorscense or other light is detected it is desirable that the different template molecules or clonal populations (e.g., amplification clusters) are physicially separated and arranged so that signals corresponding to different the templates are optically distinguishable. Sequences of tagged nucleic acid molecules of the disclosure may be determined using such methods. Exemplary approaches for sequencing include sequencing on beads and sequencing on a planar substrate.

### 6.0 Sequencing on beads

In some approaches templates on beads are sequenced, including beads comprising clonal populations prepared as described in **Part 1.**

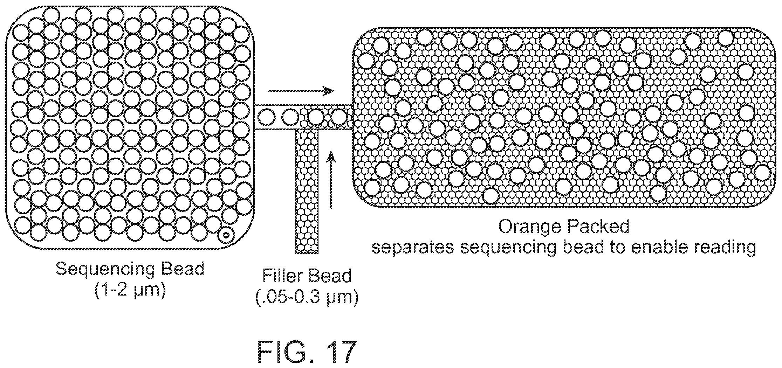

**Fig. 17** illustrates a different on-chip sequencing using bead-based sequencing reactions. As used herein, beads on which target nucleic acids or their amplification products are, or may be, immobilized are referred to as "sequencing beads." In an embodiment illustrated in **Fig. 17****,** one or more pre-sequencing steps (e.g., reverse transcription, cleavage, ligation, amplification) may occur on sequencing beads in a first chamber (shown left) and the sequencing beads may be transported to a second chamber (shown right) for the sequencing reaction and optionally, additional pre-sequencing reactions (e.g., amplification). In this context, "sequencing reaction" refers to the generation and detection of signal (typically detection of visible or fluorescent radiation) that provides nucleic acid sequence information. For example, in the case of Illumina/Solexa type sequencing, bridge amplification would be considered a pre-sequencing step, and could occur in either chamber. In some cases there may be multiple pre-sequencing chambers.

### 6.1. Use of filler beads to produce optically distinguishable signals

In the approach illustrated in **Fig. 17****,** when the sequencing beads are transported from the first to the second chamber they are 'diluted' by the introduction of "filler beads." Filler beads are "inert," in the sense that template nucleic acids are not immobilized on filler beads, and they do not produce detectable signal during the sequencing process. The effect of the addition of the filler beads is to spatially separate the sequencing beads from each other so that signals from individual sequencing beads are optically distinguishable.

Generally the sequencing beads and filler beads are roughly spherical. Although a spherical shape is not required, for purposes of simplicity, and not limitation, beads will be referred to as having 'diameters' although beads of other shapes (e.g., having a simlar volume as a sphere) are contemplated. Typically the filler beads are smaller than the sequencing beads, for example, having a diameter that is about 1/3^{rd} to 1/40^{th} the diameter of the sequencing beads. In one embodiment, the sequencing beads are about 1 to about 3 microns in diameter (e.g., about 2, such as 2.02 microns) and the filler beads are about 0.05 to about 0.4 microns in diameter (e.g., about 0.3, such as 0.28 microns). For example and not limitation, the ratio of sequencing beads to filler beads in the sequencing chamber may be in the range of 1:10⁶ to 1:10³ (numbers of beads) and/or in the range of 1:2 to 1:20 (volume of beads).

In some embodiments the packing density (the fraction of the total bead volume, or chamber volume, filled by the sequencing beads is in the range of 20% - 85%, such as 40 - 70%, such as 55% - 65%, e.g., about 60%. For illustration, a chamber 1 mm wide and 5 mm long (an area of 5 x 10⁶ square microns) accomadates 1.5 million sequencing beads at 60% packing density.

In an alternative approach the sequencing steps and the detection steps occur in the same chamber, and filler beads are introduced in to the chamber, diluting and seperating the sequencing beads, after at least one pre-sequencing step and before the detection step.

Discussed herein therein is provided a microfluidic device comprising a first, or "pre-sequencing," chamber (in which one or more pre-sequencing reactions occur) and a second, or "sequencing," chamber (suitable for sequencing and detection reactions) connected by a channel having a dimension large enough to allow the sequencing beads to travel from the first to the second chamber. In an embodiment the channel has no cross-sectional dimension (e.g., diameter, width, depth) smaller than 1 micron (e.g., a diameter 1 micron or greater) and preferably no dimension smaller than 2 microns, more preferably no dimension smaller than 3 microns. In one embodiment the dimensions of the first channel are selected to allow sequencing beads to flow though only, or primarily, in "single-file."

In some embodiments, filler beads are combined with sequencing beads before they enter the sequencing chamber, as illustrated in the figure. Thus, in one embodiment the device comprises a second microfluidic channel in fluidic communication with (a) the first channel or with the second chamber and (b) with a source of filler beads. The dimensions of the second channel are selected to allow the passage of filler beads and may be smaller than those of the first channel. In alternative embodiments, the filler beads and sequencing beads enter the sequencing chamber (i) through separate ports and/or (ii) at separate times. In one embodiment the filler beads are added first and mixing occurs when the sequencing beads are added.

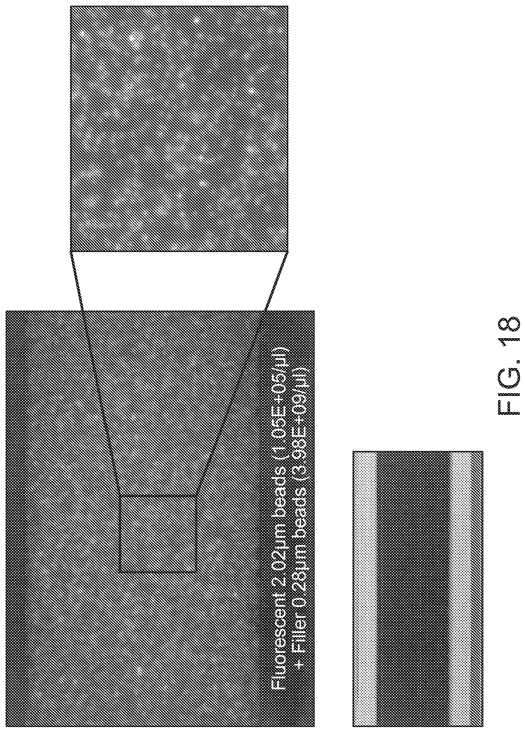

**Fig. 18** is a fluorscence image that illustrates detection of fluorscent signal from a chamber showing that signals from individual beads can be distinguished when sequencing beads (or a surrogate) are seperated by filler beads. The fluorescent beads have a diameter of 2.02 microns (1.05 x 10⁵ beads/microliter). The filler beads have a diameter of 0.28 microns (3.98 x 10⁹ beads/microliter).

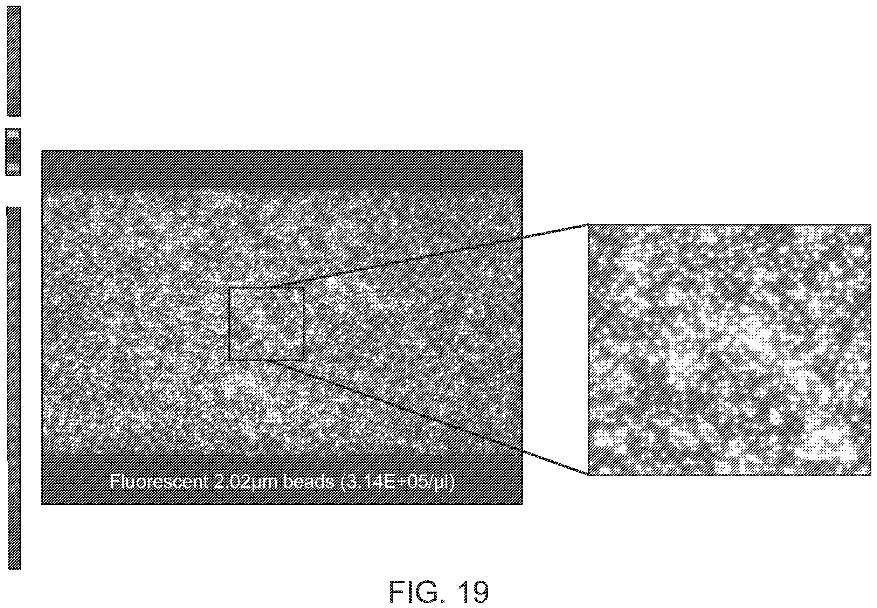

**Fig. 19** illustrates that it is more difficult, but possible, to distinguish individual sequencing beads in the absense of filler beads (3.14 x 10⁵ beads/microliter).

The dimensions of the first and second chambers may vary depending on the needs of the operator, sequencing method selected, and the method of signal detection. The size and dimensions of the first chamber will be selected based, in part, on the desired capacity to carry out the pre-amplification steps.

The size and dimensions of the second (sequencing) chamber will take into account three factors. First, generally the second chamber will be large enough to process the reaction products of the first chamber. That is, the size of the second chamber will tend to increase with the size of the first chamber. Second, the second chamber should be large enough to accommadate the filler beads, when used and/or large enough to allow for physical (and optical) separation of sequencing templates. As will be appreciates, optical separation generally requires that the beads be separated in the X-Y dimensions, rather than simply the Z dimension (where the signal detection is roughly orthoginal or incidental to the X-Y dimension). Simply put, it is difficult, for example, to distingush signal from two beads stacked in the Z plane, one above the other or other. The reference to beads that are 'optically distinguishable' captures this fact.

In one approach, the sequencing chamber accomodates only a single layer of beads. For example, the depth of the sequencing chamber may be close to the diameter of the sequencing beads.

The surface area (i.e., X-Y dimension) of the chamber may be any suitable area, such as 0.1 mm² to 50 mm². In one approach (e.g., for single cell mRNA sequencing the area may be in the range of 0.3 mm² to 6 mm², assuming about 200,000 to 5 M reads are required for appropriare coverage). In some embodiments the area is in the range 1 - 2 mm² for sequencing mRNA from a single cell.

If it is assumed there are 100-300 x 10⁵ transcripts per cell at least 1-3 million beads would be required. However, for certain applications fewer reads and fewer beads are required. For example, 200,000 reads are sufficient to differentiate cell phenotype (and possibly detect heterogeneity). AA Pollen et al., Nat Biotechnol. 2014 Oct;32(10):1053-8.

### 6.2 Minimizing movement of beads in the second chamber

In some embodiments sequencing beads and filler beads are packed tightly in the second chamber to minimize movement during the sequencing reactions (e.g., during wash steps between sequencing cycles). Movement of beads makes it more computationally challenging to interpret signals.

In one approach, after sequencing beads and filler beads are introduced into the second chamber, the beads are cross-linked to lock them in place (e.g., by exposure to a chemical or physical agent). In one embodiment only the filler beads are cross-linked to each other. Linkers, cross-linking agents, and cross-linking conditions that do not interfere with the sequencing and detection steps should be used.

Other ways to minimize bead movement is to introduce beads into nanowells, or immobilize them on a substrate within the chamber.

### 6.3 Other ways to generate optically distinguishable signals

As noted above, in one bead based method, the sequencing chamber accomodates only a single layer of beads because the depth of the chamber. In alternative bead-based approaches, (i) beads may be immobilized in spaced compartments or pads on the floor of the chamber; (ii) may be constrained by a ligand-antiligand based interaction with the chamber floor (e.g., an antiligand spotted at separate positions on the chamber floor interacts with a ligand on the bead): (iii) may be constrained by a physical interaction with the floor (for example, the floor may be patterned with negatively charged spots seperated by a hydrophobic or inert surface such that nucleic acid-covered beads are immobilized on the separated spots, In some embodiments beads are randomy distributed on the chamber floor at sufficiently low density to achieve optically separated signals. This has the obvious disadvantage of reducing capacity.

In one approach beads are introduced into a chamber comprising a substrate with at least 10,000 reaction chambers (cavities or wells) sized to accomadate a single bead (e.g., similar to a PicoTiterPlate™; see International patent publication WO 2005003375). The beads are physically separated in the wells.

### 6.4 Sequencing modules

The combination of the first and second chambers, optionally a source of filler beads, and connecting channels) may be referred to as a "sequencing module." As illustrated, cells may be captured, wahed, and lysed in the microfluidic device outside the sequencing module, followed by introduction of the cell lysate (or an RNA containing fraction) into the first chamber of the module. First strand cDNA synthesis, cleavage and ligation of the adaptor oligonucleotide may be carried out in the first chamber.

In one embodiment mRNA and beads are combined before entry into the "first" chamber, for example, RNA may be captured in a bead column or 'pre-chamber' and the beads then transferred to the 'first chamber.'

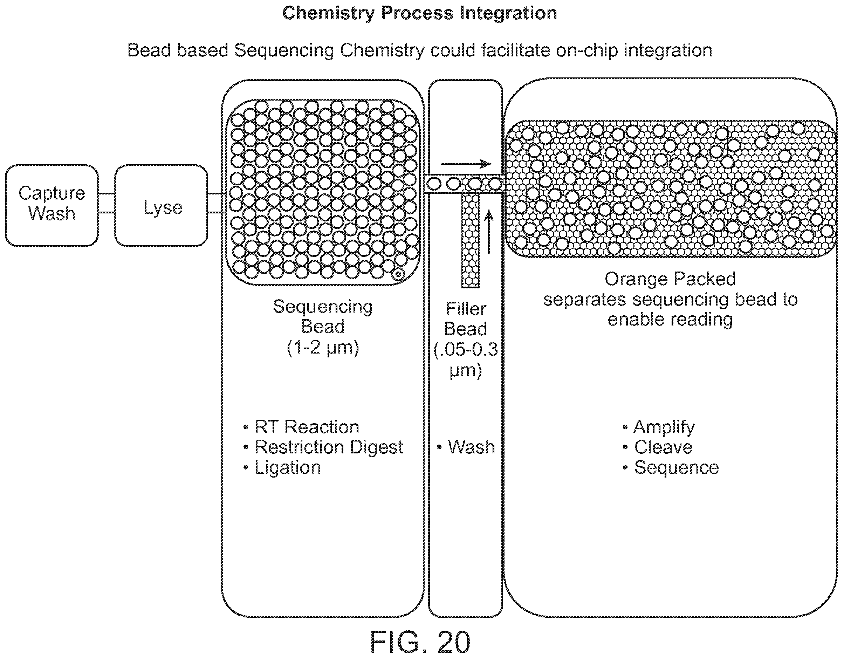

As illustrated in **Fig. 20****,** amplification and sequencing/imaging may be carried out In the second chamber.

### 6.5 Embodiments not using beads

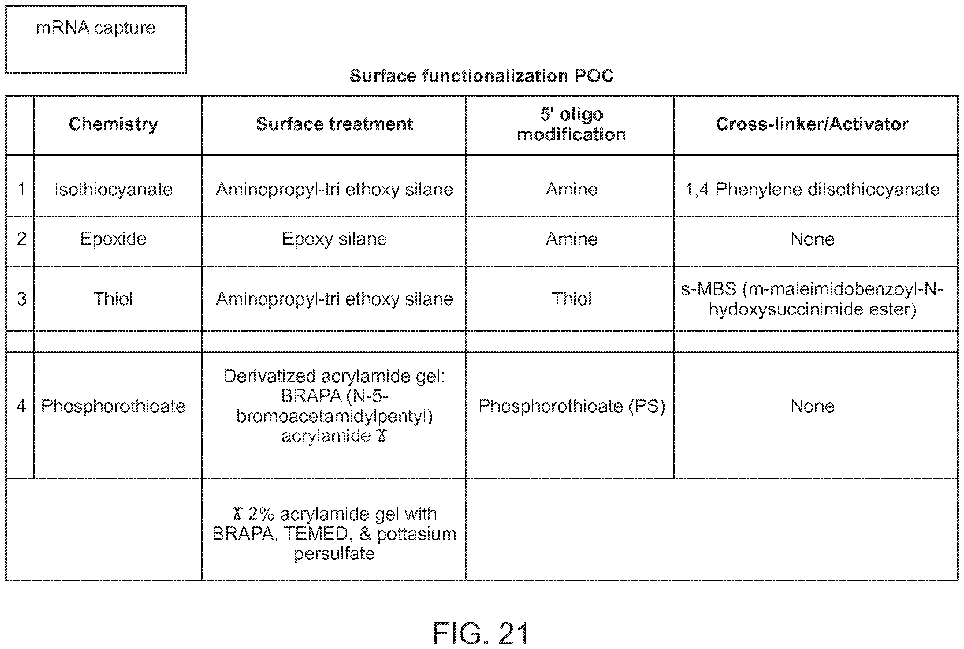

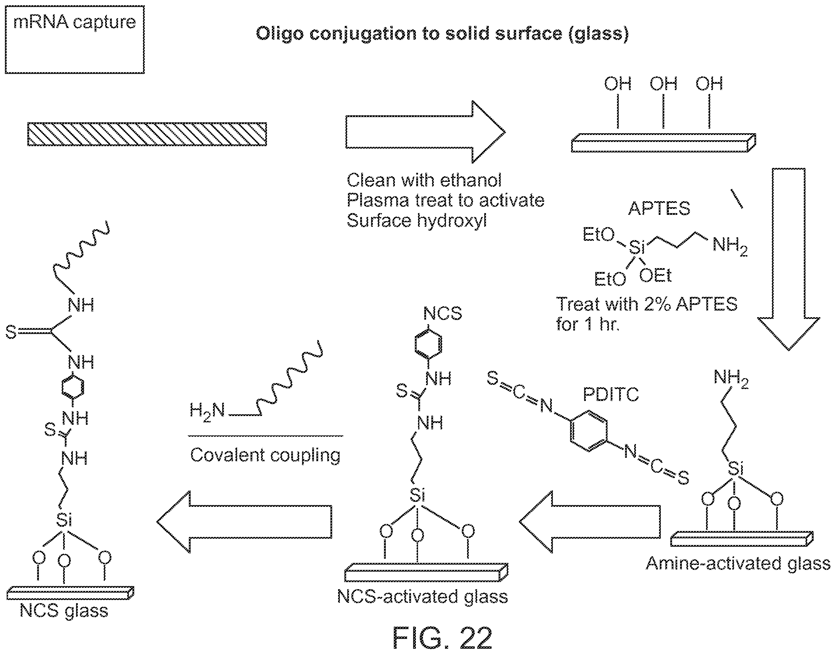

In some embodiments, RNA or DNA templates that are not immobilized on beads are transported into the sequencing chamber and are immobilized on a substantially planar substrate. In embodiments the is glass or PDMS on, or comprised by, the chamber floor. A number of approaches to such immobilization are known and could be adapted to the present disclosure. In one approach a cell lysate is contacted with a poly d(T) coated surface followed by reverse transcription and cDNA sequencing. **Figs. 21** **and** **22** show exemplary methods for immobilizing oligonucleotides to a surface and may other methods are know in the art. In an approach RNA is introduced into the chamber containing a lawn of capture oligonucleotides at low enough density that individual RNA molecules (and consequently, clonal populations derived fro the RNAs) are physically seperated and signals eminating therefrom are optically distinguishable. Methods for making random or ordered arrays of template for sequencing are well known. See, for example, US Pat. Pub. 2013/0118153; C. Adessi et al., Nucleic Acids Res. 2000 Oct 15;28(20):E87; M. Fedurco et al., Nucleic Acids Res. 2006 Feb 9;34(3):e22

In one embodiment, cell lysis and RNA capture take place in the same chamber.

### 6.6 Performing multiple cycles

The sequencing-by-synthesis reaction involves multiple cycles of incorporation of a nucleotide or nucleotide analog and detection of signal. This is typically carried out by introducing reagents into the sequencing chamber at a point in the cycle, and removing the reagents and products prior to the beginning of a subsequent cycle. This is accomplished by introducing reagents, reagent solutions, wash solution, and the like into the chamber, and removing them using standard microfluidic methods, In some embodiments, the microfluidic device is preloaded with sequencing reagents and/or wash solutions prior to sequencing.

### 6.7 Imaging and Analysis

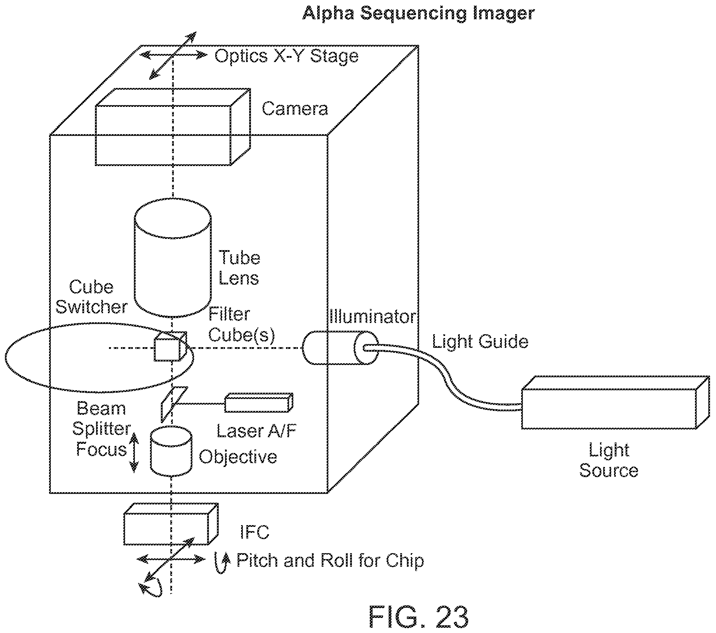

Signal from the second chamber can be collected using a camera (e.g., CCD camera) and optical systems developed by Fluidigm Corp. and known in the art. See, e.g, Fig. 23. In such embodiments, the material between the camera and the signal origin will be transparent to the signal.

In other embodiments, wignal detection may rely on fiber optic or other sensors associated with a particular bead or well.

### PART 4: INTEGRATED MICROFLUIDIC DEVICES

### 7. Integrated Devices

**Fig. 20** illustrates one way a sequencing cassette can be integrated into a microfluidic chip. In the approach shown the following steps may be carried out, in which steps 2-9 (and optionally step 1) are carried out in the microfluidic device, and steps 6-9 are carried out in the sequencing module:

**TABLE 2**

| Step | |
|---|---|
| 1 | Enrich |
| 2 | Load and capture single cells |
| 3 | Wash and optionally stain cells |
| 4 | Optionally image captured cells |
| 5 | Lyse cells |
| 6 | Capture mRNA on substrate (e.g., beads) |
| 7 | Synthesize cDNA (e.g., cDNA synthesis, cleavage, adaptor ligation |
| 8 | Clonal amplification |
| 9 | Sequence (e.g., SBS) |
| 10 | Analyse |

It will be recognized that **Fig. 20** and **Table 2** are for illustration and not limitation, and that numerous variations of the process are possible.

### 7.1 Cell Enrichment

Cell enrichment may occur within the microfluidic device, "off-chip," or both. Enrichment parameters include physical properties (e.g., size, deformaty, density, charge) and biological properties (e.g., expression of marker proteins.

### 7.2 Capture of Single Cells

Capture of single cells may be carried out using a variety of method. In one approach, a single cell capturing microfluidic device having features described in WO-2013/130714 ("Methods, systems, and devices for multiple single-cell capturing and processing using microfluidics") is used to isolate individual cells, process and sequence nucleic acids. It will be within the ability of one skilled in the art guided by this specification to make certain modifications, if desired, such as, for example, incorporating a sequencing module as described above. In one approach a single cell capturing microfluidic device having features described in WO-2014/144789 ("Methods and devices for analysis of defined multicellular combinations") is used to isolate individual cells, process and sequence nucleic acids. It will be within the ability of one skilled in the art guided by this specification to make certain modifications, if desired, such as, for example, incorporating a sequencing module as described above.

### PART 5: ADDITIONAL FEATURES

### 8. Additional Features

This section provides additional description about certain elements described above.

### 8.1 Anchor polynucleotides

The anchor polynucleotides provided herein are used to capture mRNA molecules to a solid support. Anchor polynucleotides may therefore include an oligo d(T) primer to capture mRNA molecules. The anchor polynucleotide may further provide means to amplify a target polynucleotide (e.g., cDNA) after it has been tagged with the adapter nucleic acid. The anchor polynucleotide may further include a restriction enzyme recognition sequence to provide means for removal of the immobilized target polynucleotide from the solid support after amplification and/or sequencing. Examples, for illustration and not limitation, of anchor polynucleotides are illustrated in Fig. 2. In this embodiment, two anchor polynucleotides attached to a solid support (bead) are shown. One anchor polynucleotide, referred to herein as "first anchor polynucleotide" includes an amplification primer (AP1) and a restriction recognition site (e.g., cut site 1 or CS1). The anchor polynucleotide including an oligo d(T) primer, an amplification primer complementary to AP1 (AP1') and a restriction recognition site (e.g., cut site 2 or CS2) is referred to herein as "second anchor polynucleotide." Further examples of anchor polynucleotides are illustrated in **Fig. 3. Fig. 3** shows a first and a second anchor polynucleotide, wherein a mRNA template is annealed via its polyA tail to the oligo d(T) of the second anchor polynucleotide.

### 8.2 First anchor polynucleotide

In embodiments, a first anchor polynucleotide is immobilized on the solid support. In embodiments, the first anchor polynucleotide includes a first amplification nucleic acid sequence and serves as an amplification primer (also referred to as "amplification primer 1" or "AP1"). In embodiments, the first anchor polynucleotide includes a first release nucleic acid sequence such as a restriction enzyme recognition site (also referred to as "cut site 1" or "CS1"). In embodiments, the first release nucleic acid sequence (e.g., CS1) connects the first amplification nucleic acid sequence (e.g., AP1) to the solid support.

### 8.3 Second anchor polynucleotide

In some embodiments a second anchor polynucleotide is immobilized on the solid support. In embodiments, the second anchor polynucleotide includes a second amplification nucleic acid sequence (also referred to as amplification primer 2 or AP2). In embodiments, the second anchor polynucleotide includes a second release nucleic acid sequence such as a restriction enzyme recognition site (also referred to as cut site 2 or CS2). In embodiments, the second release nucleic acid sequence (e.g., CS2) connects the second amplification nucleic acid sequence (e.g., AP2) to the solid support. In embodiments, the second amplification nucleic acid sequence (e.g., AP2) connects the second release nucleic acid sequence (e.g., CS2) to the target polynucleotide capturing sequence (e.g., oligo dTT²⁰). Thus, the single stranded cDNA as provided herein may be immobilized on the solid support by being covalently attached to the deoxy-thymine sequence (e.g., oligo dTT²⁰), wherein the deoxy-thymine sequence is linked to the second amplification nucleic acid sequence (e.g., AP2), which is bound to the solid support through the second release nucleic acid sequence (e.g., CS2).

As describe above, the target polynucleotide may be a single stranded DNA (e.g., cDNA). Where the target polynucleotide is a cDNA, the target polynucleotide may be linked to the solid support through a second anchor polynucleotide. The second anchor polynucleotide includes a target polynucleotide capturing sequence. In embodiments, the target polynucleotide capturing sequence is a deoxy-thymine sequence, also referred to herein as oligo d(T)₂₀.

Where the target polynucleotide is an RNA (target ribonucleic acid), the target ribonucleic acid may be immobilized on a solid support through hybridization to a target polynucleotide capturing sequence (e.g., an oligo d(T)₂₀). As described above, the target polynucleotide capturing sequence may form part of a second anchor polypeptide provided herein. Where the target polynucleotide capturing sequence is oligo d(T)₂₀, the target ribonucleic acid hybridizes through its polyadenylated 3' end to the target polynucleotide capturing sequence.

### 8.4 Adapter nucleic acid

In the methods provided herein, an adapter nucleic acid sequence is ligated to the cleaved target polynucleotide, thereby forming a tagged nucleic acid sequence. The adapter nucleic acid sequence as provided herein may be any nucleic acid capable of being ligated to the cleaved target polynucleotide (e.g., cDNA). The adapter nucleic acid sequence includes a primer amplification sequence therefore provides for means of amplification of the target polynucleotide. In an embodiment, the adapter nucleic acid includes an amplification primer complement (AP1'), which may be used to anneal to the amplification primer (AP1) of the first anchor polynucleotide, thereby providing the means for amplification of the target polynucleotide by, e.g., bridge PCR.

In embodiments, the adapter nucleic acid includes an amplification primer complement (AP1'), which may be annealed to an amplification primer (AP1), which is not attached to the solid support, but added to the reaction solution, thereby allowing for amplification of the target polynucleotide by isothermal template, also referred to herein as wildfire PCR.

In embodiments, the adapter nucleic acid sequence is a double stranded nucleic acid. In embodiments, the adapter nucleic acid sequence is a single stranded nucleic acid. in embodiments, the adaptor nucleic acid sequence includes a first amplification nucleic acid sequence complement. A first amplification nucleic acid sequence complement is a nucleic acid sequence specifically complementary to the first amplification nucleic acid sequence described above. The terms "first amplification nucleic acid sequence" and "second amplification nucleic acid sequence" as provided herein refer to isolated nucleic acids that recognize a target nucleic acid sequence (first and second amplification nucleic acid sequence complement). The first and second amplification nucleic acid sequences are short nucleic acid molecules, for instance DNA oligonucleotides 10 nucleotides or more in length. A contiguous complementary oligonucleotide (e.g., a first amplification nucleic acid sequence complement or a second amplification nucleic acid sequence complement) may be annealed through hybridization to the first and/or second amplification nucleic acid sequence. The contiguous complementary oligonucleotide may be extended along the target polynucleotide by a DNA polymerase enzyme using PCR or other nucleic-acid amplification methods known in the art, thereby amplifying the target polynucleotide. In embodiments, the first and second amplification nucleic acid sequence are independently about 15, 20, 25, 30 or 50 nucleotides or more in length. In embodiments, the first amplification nucleic acid sequence and the second amplification nucleic acid sequence are independently about 10 to about 100 nucleotides in length, in embodiments, the first amplification nucleic acid sequence and the second amplification nucleic acid sequence are independently about 15 to about 95 nucleotides in length

Where the adaptor nucleic acid sequence includes a first amplification nucleic acid sequence complement, the first amplification nucleic acid sequence complement may hybridize to a first amplification nucleic acid sequence. As described above, the first amplification nucleic acid sequence is also referred to herein as amplification primer 1, or AP1 and forms part of a first anchor polynucleotide, which is immobilized to the solid support. In the methods provided herein the first anchor polynucleotide may be covalently bound to the solid support. In embodiments, the first amplification nucleic acid sequence complement is hybridized to the first amplification nucleic acid sequence under conditions allowing for PCR amplification, thereby amplifying the target polynucleotide (i.e. tagged nucleic acid sequence). In embodiments, after the ligating of step (iv) the tagged nucleic acid sequence is contacted with a first amplification nucleic acid sequence under conditions allowing for PCR amplification. In embodiments, the first amplification nucleic acid sequence is at least partially complementary to the first amplification nucleic acid sequence complement. In embodiments, the first amplification nucleic acid is not attached to the solid support. In further embodiments, the first amplification nucleic acid hybridizes to the first amplification nucleic acid.

### 8.5 Array of tagged polynucleotides

A person of ordinary skill in the art will immediately recognize that the methods of tagging a nucleic acid sequence as provided herein may be applicable to tag a plurality of nucleic acid sequences. Where the method provided herein includes tagging a plurality of nucleic acid sequences, each of the target polynucleotides may be independently different. Therefore, the target polynucleotides may be heterogeneous. In embodiments, the plurality of target polynucleotides is a plurality of cDNA sequences. In embodiments, the plurality of target polynucleotides is a plurality of ribonucleic acid sequences. The plurality of target polynucleotides may be derived from an isolated cell. An isolated cell as provided herein is a cell that has been substantially separated or purified away from other components (cells) in a cell culture, tissue, organ or organism in which the cell previously occurred. Cells that have been "isolated" include cells purified by standard purification methods.

In one aspect, a method of forming a plurality of tagged heterogeneous polynucleotides, is provided. According to the method (i) a plurality of heterogeneous target polynucleotides is immobilized on a solid support, thereby forming a plurality of immobilized heterogeneous target polynucleotides. (ii) A plurality of heterogeneous recognition-oligonucleotides is hybridized to the immobilized heterogeneous target polynucleotides, thereby forming a plurality of recognition-oligonucleotide-target polynucleotide hybrids, (iii) The recognition-oligonucleotide-target polynucleotide hybrids are cleaved with a cleaving agent, thereby forming a plurality of cleaved recognition-oligonucleotide-cleaved target polynucleotide hybrids, (iv) An adapter nucleic acid sequence is ligated to the plurality of cleaved target polynucleotides, thereby forming a plurality of tagged heterogeneous polynucleotides. As described above the same definitions apply to the aspects of forming a plurality of tagged heterogeneous polynucleotides including embodiments, thereof. For example, the solid support may be a bead structure. The plurality of heterogeneous target polynucleotides may be single stranded cDNA sequences. The cleaving agent may be a restriction enzyme.

### 8.6 Embodiments of cDNA tagging

As described above the target polynucleotide may be a cDNA. Thus, in one aspect a method of forming a tagged single stranded cDNA is provided. According to the method (i) a target cDNA is immobilized on a solid support, thereby forming an immobilized target cDNA. (ii) A recognition-oligonucleotide is hybridized to the immobilized target cDNA, thereby forming a recognition-oligonucleotide-cDNA hybrid, (iii) The recognition-oligonucleotide-cDNA hybrid is cleaved with a cleaving agent, thereby forming a cleaved recognition-oligonucleotide-cleaved cDNA hybrid. (iv) An adapter nucleic acid is ligated to the cleaved cDNA, thereby forming a tagged single stranded cDNA Where the target polynucleotide is a cDNA, the cDNA may be immobilized on the solid support using immobilization methods commonly known in the art and as described above. For example, the cDNA may be directly immobilized to a chemically modified (functionalized) solid support by covalent attachment, In other embodiments, the cDNA is attached to the solid support through a second anchor polynucleotide as described above. Where the cDNA is attached to the solid support through a second anchor polynucleotide, an mRNA molecule is hybridized on a solid support by hydrogen bonding between the polyadenylated 3' end of the mRNA and the nucleic acid sequence of a target polynucleotide capturing sequence (e.g., deoxy-thymine sequence or oligo dTT²⁰), thereby forming an immobilized mRNA. As described above the target polynucleotide capturing sequence may form part of a second anchor polypeptide. The immobilized mRNA is subsequently reverse transcribed, thereby forming an RNA:DNA hybrid. The mRNA of the RNA:DNA hybrid may be degraded by contacting the hybrid with a endoribonuclease enzyme (e.g., RNAse H), thereby forming a single stranded cDNA attached on a solid support through a target polynucleotide capturing sequence. The immobilized single stranded cDNA (target cDNA) may be hybridized to a recognition-oligonucleotide as described above, thereby forming a recognition-oligonucleotide-cDNA hybrid. As described above Recognition Oligonucleotide may include a cleaving agent recognition sequence (e.g. a BaeG1 recognition sequence) flanked by degenerate nucleic acid sequences. The recognition-oligonucleotide-cDNA hybrid may be cleaved with a cleaving agent (e.g., BaeG1), thereby forming a cleaved recognition-oligonucleotide-cleaved cDNA hybrid. As described above the cleaved recognition-oligonucleotide-cleaved cDNA hybrid may include a 5' overhang. An adapter nucleic acid as described above is ligated to the cleaved cDNA, thereby forming a tagged single stranded cDNA. Any ligation method and DNA ligase commonly known in the art may be used to ligate the adapter nucleic acid to the cleaved cDNA.

### 8.7 Embodiments of RNA tagging

As described above the target polynucleotide may be a ribonucleic acid. Thus, in another aspect, a method of forming a tagged nucleic acid sequence is provided. According to the method (i) a target ribonucleic acid is immobilized on a solid support, thereby forming an immobilized target ribonucleic acid. (ii) The immobilized target ribonucleic acid is reverse transcribed, thereby forming an RNA:DNA hybrid, (iii) The RNA:DNA hybrid is cleaved with an RNA:DNA cleaving agent, thereby forming a cleaved RNA:DNA hybrid. (iv) An adapter nucleic acid sequence is ligated to the cleaved RNA:DNA hybrid. (v) The ribonucleic acid sequence is removed from the RNA:DNA hybrid, thereby forming a tagged nucleic acid sequence. Where a target ribonucleic acid is immobilized on a solid support, the target ribonucleic acid may be an mRNA and the immobilization may be performed as described above through hydrogen bonding between the polyadenylation sequence of the mRNA and the polynucleotide capturing sequence described herein. By reverse transcription of the mRNA an RNA:DNA hybrid is formed and the RNA:DNA hybrid may be cleaved using a cleaving agent. The cleaving agent may be a restriction endonuclease capable of cleaving double-stranded hybrids of DNA and RNA, wherein one strand is a DNA and the other strand is a RNA. Upon cleavage of the RNA:DNA hybrid a 5' overhang, 3' overhang or blunt ends without overhang may be generated. Therefore, the cleaved RNA:DNA hybrid may include a 5' overhang, 3' overhang or blunt ends and may subsequently be ligated to an adapter nucleic acid. Once the adapter nucleic acid has been ligated to the RNA:DNA hybrid, the RNA may be removed by digestion using an endoribonuclease as described above, resulting in the formation of a tagged nucleic acid sequence.

A person of ordinary skill in the art will immediately recognize that the methods of tagging a nucleic acid sequence as provided herein may be applicable to tag a plurality of nucleic acid sequences. Thus, in another aspect a method of forming a plurality of tagged heterogeneous nucleic acid sequences is provided. According to the method (i) a plurality of heterogeneous target ribonucleic acid sequences are immobilized on a solid support, thereby forming a plurality of immobilized heterogeneous target ribonucleic acid sequences, (ii) The immobilized heterogeneous target ribonucleic acid sequences are reverse transcribed, thereby forming a plurality of heterogeneous RNA:DNA hybrids. (iii) The plurality of heterogeneous RNA:DNA hybrids are cleaved with an RNA:DNA cleaving agent, thereby forming a plurality of cleaved RNA:DNA hybrids. (iv) An adapter nucleic acid sequence is ligated to the plurality of cleaved RNA:DNA hybrids and (v) the ribonucleic acid sequences are removed from the cleaved RNA:DNA hybrids, thereby forming a plurality of tagged heterogeneous nucleic acid sequences.

### 8.8 Recognition-Oligonucleotide Libraries

In another aspect, a library of recognition-oligonucleotides including a plurality of heterogeneous recognition-oligonucleotides each including a restriction enzyme recognition sequence flanked by degenerate nucleic acid sequences is provided. The degenerate nucleic acid sequences as provided herein flank the restriction enzyme recognition sequence (also referred to herein as cleaving agent recognition sequence) and include degenerate nucleotides. The degenerate nucleotides may be complementary or partially complementary to different target polynucleotides (e.g. single stranded cDNA). The term "partially complementary" refers to a recognition-oligonucleotide which is capable of hybridzing to more than target polynucleotide, wherein each target polynucleotide is different. In embodiments, the cleaving agent recognition sequence is flanked by degenerate nucleic acid sequences. In embodiments, the degenerate nucleic acid sequences are partially complementary to a target polynucleotide (e.g., a cDNA). In embodiments, the degenerate nucleic acid sequences are specifically complementary to a target polynucleotide. In embodiments, the recognition-oligonucleotides have a structure of 5' Aₙ-Xₘ-Bₙ 3', wherein A and B are nucleotide sequences complementary or partially complementary to a sequence comprised by a target polynucleotide and n is independently an integer from 10-40. X is a cleaving agent recognition sequence and m is an integer from 4 to 10. The cleaving agent may be a restriction enzyme as described above (e.g., BaeG1). In embodiments, the library forms part of a microfluidic device.

### 8.9 PCR Amplification

The tagged polynucleotides provided herein may be amplified and subsequently sequenced. Any nucleic acid amplification method known in the art may be used. In one specific, non-limiting example, polymerase chain reaction (PCR) is used to amplify the tagged polynucleotides provided herein. In embodiments, the tagged polynucleotides provided herein are amplified using bridge PCR. Thus, in embodiments, the PCR amplification is bridge PCR. The technique of bridge PCR is well known in the art and has been described for example in published international application WO2013/131962 A1. In embodiments, the tagged polynucleotides provided herein are amplified using isothermal template walking. Thus, in embodiments, the PCR amplification is isothermal template walking. Isothermal template walking is an amplification method well known in the art and is described for example by Ma Z et al., PNAS 2013;110:14320-14323. In embodiments, the method includes after the contacting of step sequencing the amplified cDNA. In embodiments, each step occurs in a microfluidic device. Examples of a microfluidic device are disclosed in published US application number US2013/0302883, US2013/0302884, US2013/0296196, US2013/0295602, and US2013/0302807.

In another aspect, a method of amplifying a cDNA sequence is provided. According to the method (i) an RNA molecule extracted from an isolated cell is simmobilized on a solid support, thereby forming an immobilized ribonucleic acid sequence. (ii) The immobilized ribonucleic acid sequence is reverse transcribed, thereby forming an immobilized RNA:DNA hybrid, (iii) The ribonucleic acid sequence is removed from the RNA:DNA hybrid, thereby forming an immobilized cDNA sequence. (iv) A recognition-oligonucleotide is hybridized to the immobilized cDNA sequence, thereby forming a recognition- oligonucleotide-cDNA hybrid, (v) The recognition- oligonucleotide-cDNA hybrid is cleaved with a cleaving agent, thereby forming a cleaved recognition- oligonucleotide-cleaved cDNA hybrid, (vi) An adapter nucleic acid sequence is ligated to the cleaved cDNA, thereby forming a tagged cDNA sequence. (vii) The tagged cDNA sequence is hybridized to an amplification nucleic acid sequence under conditions allowing for PCR amplification, thereby amplifying a cDNA sequence. In embodiments, amplification nucleic acid sequence is covalently bound to the solid support. In embodiments, the amplified cDNA is sequenced after the hybridizing of step (vii). Any sequencing method known in the art may be used for sequencing the amplified cDNA

In another aspect, a method of amplifying a cDNA sequence is provided. According to the method (i) an RNA molecule extracted from an isolated cell is immobilized on a solid support, thereby forming an immobilized ribonucleic acid sequence. (ii) The immobilized ribonucleic acid sequence is reversed transcribed, thereby forming an immobilized RNA:DNA hybrid, (iii) The RNA:DNA hybrid is cleaved with an RNA:DNA cleaving agent, thereby forming a cleaved RNA:DNA hybrid, (iv) An adapter nucleic acid sequence is ligated to the cleaved RNA:DNA hybrid, (v) The ribonucleic acid is removed from the cleaved RNA:DNA hybrid, thereby forming a tagged cDNA sequence and (vi) the tagged cDNA sequence is contacted with an amplification nucleic acid sequence under conditions allowing for PCR amplification, thereby amplifying said cDNA sequence. In embodiments, the amplification nucleic acid sequence is covalently bound to the solid support (e.g. AP1). In embodiments, the amplified cDNA is sequenced after the contacting of step (vi). In embodiments, the PCR amplification is PCR bridge amplification. In embodiments, the PCR amplification is isothermal template walking. In embodiments, the single cell is isolated from a heterogeneous population of isolated cells. In embodiments, each step of the methods provided herein occurs in a microfluidic device.

Citation of publications and patent documents (patents, published patent applications, and unpublished patent applications) is not intended as an admission that any such document is pertinent prior art, nor does it constitute any admission as to the contents or date of the same.

## Claims

1. A method of forming a tagged nucleic acid sequence, said method comprising:
(i) immobilizing a target polynucleotide on a solid support, comprising
(a) capturing a RNA molecule to a solid support, thereby forming a captured RNA,
(b) reverse transcribing said captured RNA, then removing the captured RNA, thereby forming a target polynucleotide immobilized to said solid support thereby forming an immobilized target polynucleotide cDNA;
(ii) hybridizing a recognition-oligonucleotide to said immobilized target polynucleotide, thereby forming a recognition-oligonucleotide-target polynucleotide hybrid;
(iii) cleaving said recognition-oligonucleotide-target polynucleotide hybrid with a cleaving agent selected from a restriction enzyme thereby forming a cleaved recognition-oligonucleotide-cleaved target polynucleotide hybrid comprising a cleaved target polynucleotide; and
(iv) ligating an adapter nucleic acid sequence to said cleaved target polynucleotide, thereby forming a tagged nucleic acid sequence;
wherein a first anchor polynucleotide comprising a first amplification nucleic acid sequence is covalently bound to said solid support, and
wherein said adapter nucleic acid sequence comprises a first amplification nucleic acid sequence complement of said first anchor polynucleotide..

2. The method of the preceding claim, wherein said RNA molecule is extracted from an isolated cell.

3. The method of any preceding claim, wherein said solid support comprises a bead structure, optionally wherein said bead structure is a biotin bead.

4. The method of any preceding claim, wherein said first anchor polynucleotide comprising a first amplification nucleic acid sequence further comprises a first release nucleic acid sequence, optionally wherein said first release nucleic acid sequence connects said first amplification nucleic acid sequence to said solid support, optionally wherein said first release nucleic acid sequence comprises a restriction enzyme cleavage sequence.

5. The method of claim 1, comprising hybridizing said first amplification nucleic acid sequence complement to said first amplification nucleic acid sequence under conditions allowing for PCR amplification, thereby amplifying said tagged nucleic acid sequence.

6. A method of claim 1 wherein the tagged nucleic acid sequence is a plurality of tagged heterogeneous polynucleotides, said method comprising:
(i) immobilizing a plurality of heterogeneous target polynucleotides on a solid support as set out in step (i) (a) and (b) of claim 1, thereby forming a plurality of immobilized heterogeneous target polynucleotides;
(ii) hybridizing a plurality of heterogeneous recognition-oligonucleotides to said immobilized heterogeneous target polynucleotides, thereby forming a plurality of recognition-oligonucleotide-target polynucleotide hybrids;
(iii) cleaving said recognition-oligonucleotide-target polynucleotide hybrids with a cleaving agent selected from a restriction enzyme, thereby forming a plurality of cleaved recognition-oligonucleotide-cleaved target polynucleotide hybrids; and
(iv) ligating an adapter nucleic acid sequence to said plurality of cleaved target polynucleotides, thereby forming a plurality of tagged heterogeneous polynucleotides,
wherein said adapter nucleic acid comprises at least a first amplification nucleic acid sequence complement of a first anchor polynucleotide covalently bound to a solid support.

7. The method of claim 6, wherein said solid support comprises a bead structure.

8. A method of amplifying a cDNA sequence, said method comprising:
(i) immobilizing a target polynucleotide on a solid support, comprising
(a) capturing a RNA molecule to a solid support, thereby forming a captured RNA,
(b) reverse transcribing said captured RNA, then removing the captured RNA, thereby forming a target polynucleotide immobilized to said solid support thereby forming an immobilized target polynucleotide cDNA;
(ii) hybridizing a recognition-oligonucleotide to said immobilized target polynucleotide, thereby forming a recognition-oligonucleotide-target polynucleotide hybrid;
(iii) cleaving said recognition-oligonucleotide-target polynucleotide hybrid with a cleaving agent selected from a restriction enzyme, thereby forming a cleaved recognition-oligonucleotide-cleaved target polynucleotide hybrid comprising a cleaved target polynucleotide;
(iv) ligating an adapter nucleic acid sequence to said cleaved target polynucleotide, thereby forming a tagged cDNA sequence,
wherein a first anchor polynucleotide comprising a first amplification nucleic acid sequence is covalently bound to said solid support, and
wherein said adapter nucleic acid sequence comprises a first amplification nucleic acid sequence complement of the first anchor polynucleotide; and
(v) amplifying the cDNA sequence by clonal amplification.

9. The method of any of claims 1-8, wherein each step is performed in a microfluidic device.

10. The method of claim 8, wherein said target polynucleotide is a plurality of nucleic acid sequences each of target polynucleotides being independently different, the plurality of target polynucleotides derived from an isolated cell.

11. The method of any one of claims 1 to 10, further comprising the step of
- amplifying the tagged nucleic acid sequence by bridge amplification comprising hybridising the first amplification nucleic acid sequence complement to a first amplification nucleic acid sequence of the first anchor polynucleotide; and
- sequencing the tagged nucleic acid sequence using sequencing by synthesis.

12. The method of claim 8, further comprising the step of sequencing the amplification product of step (v).

## Patentansprüche

1. Ein Verfahren zum Bilden einer markierten Nukleinsäuresequenz, wobei das Verfahren Folgendes beinhaltet:
(i) Immobilisieren eines Zielpolynukleotids auf einem festen Träger, Folgendes beinhaltend:
(a) Einfangen eines RNA-Moleküls auf einem festen Träger, wodurch eine eingefangene RNA gebildet wird,
(b) reverses Transkribieren der eingefangenen RNA, dann Entfernen der eingefangenen RNA, wodurch ein Zielpolynukleotid gebildet wird, das auf dem festen Träger immobilisiert ist, wodurch eine immobilisierte Zielpolynukleotid-cDNA gebildet wird;
(ii) Hybridisieren eines Erkennungsoligonukleotids an das immobilisierte Zielpolynukleotid, wodurch ein Erkennungsoligonukleotid-Zielpolynukleotid-Hybrid gebildet wird;
(iii) Spalten des Erkennungsoligonukleotid-Zielpolynukleotid-Hybrids mit einem Spaltmittel, das aus einem Restriktionsenzym ausgewählt ist, wodurch ein Gespaltenes-Erkennungsoligonukleotid-gespaltenes-Zielpolynukleotid-Hybrid gebildet wird, der ein gespaltenes Zielpolynukleotid beinhaltet; und
(iv) Ligieren einer Adapternukleinsäuresequenz an das gespaltene Zielpolynukleotid, wodurch eine markierte Nukleinsäuresequenz gebildet wird;
wobei ein erstes Ankerpolynukleotid, das eine erste Amplifikationsnukleinsäuresequenz beinhaltet, kovalent an den festen Träger gebunden ist, und
wobei die Adapternukleinsäuresequenz ein erstes Amplifikationsnukleinsäuresequenzkomplement des ersten Ankerpolynukleotids beinhaltet.

2. Verfahren gemäß dem vorhergehenden Anspruch, wobei das RNA-Molekül aus einer isolierten Zelle extrahiert wird.

3. Verfahren gemäß einem der vorhergehenden Ansprüche, wobei der feste Träger eine Perlenstruktur beinhaltet, wobei die Perlenstruktur optional eine Biotinperle ist.

4. Verfahren gemäß einem der vorhergehenden Ansprüche, wobei das erste Ankerpolynukleotid, das eine erste Amplifikationsnukleinsäuresequenz beinhaltet, ferner eine erste Freisetzungsnukleinsäuresequenz beinhaltet, wobei die erste Freisetzungsnukleinsäuresequenz optional die erste Amplifikationsnukleinsäuresequenz mit dem festen Träger verbindet, wobei die erste Freisetzungsnukleinsäuresequenz optional eine Restriktionsenzymspaltungssequenz beinhaltet.

5. Verfahren gemäß Anspruch 1, das das Hybridisieren des ersten Amplifikationsnukleinsäuresequenzkomplements an die erste Amplifikationsnukleinsäuresequenz unter Bedingungen, die PCR-Amplifikation gestatten, beinhaltet, wodurch die markierte Nukleinsäuresequenz amplifiziert wird.

6. Verfahren gemäß Anspruch 1, wobei die markierte Nukleinsäuresequenz eine Vielzahl von markierten heterogenen Polynukleotiden ist, wobei das Verfahren Folgendes beinhaltet:
(i) Immobilisieren einer Vielzahl von heterogenen Zielpolynukleotiden auf einem festen Träger, wie in Schritt (i) (a) und (b) in Anspruch 1 dargelegt, wodurch eine Vielzahl von immobilisierten heterogenen Zielpolynukleotiden gebildet wird;
(ii) Hybridisieren einer Vielzahl von heterogenen Erkennungsoligonukleotiden an die immobilisierten heterogenen Zielpolynukleotide, wodurch eine Vielzahl von Erkennungsoligonukleotid-Zielpolynukleotid-Hybriden gebildet wird;
(iii) Spalten der Erkennungsoligonukleotid-Zielpolynukleotid-Hybride mit einem Spaltmittel, das aus einem Restriktionsenzym ausgewählt ist, wodurch eine Vielzahl von Gespaltenes-Erkennungsoligonukleotid-gespaltenes-Zielpolynukleotid-Hybriden gebildet wird; und
(iv) Ligieren einer Adapternukleinsäuresequenz an die Vielzahl von gespaltenen Zielpolynukleotiden, wodurch eine Vielzahl von markierten heterogenen Polynukleotiden gebildet wird,
wobei die Adapternukleinsäure mindestens ein erstes Amplifikationsnukleinsäuresequenzkomplement eines ersten Ankerpolynukleotids, das kovalent an einen festen Träger gebunden ist, beinhaltet.

7. Verfahren gemäß Anspruch 6, wobei der feste Träger eine Perlenstruktur beinhaltet.

8. Ein Verfahren zum Amplifizieren einer cDNA-Sequenz, wobei das Verfahren Folgendes beinhaltet:
(i) Immobilisieren eines Zielpolynukleotids auf einem festen Träger, Folgendes beinhaltend:
(a) Einfangen eines RNA-Moleküls auf einem festen Träger, wodurch eine eingefangene RNA gebildet wird,
(b) reverses Transkribieren der eingefangenen RNA, dann Entfernen der eingefangenen RNA, wodurch ein Zielpolynukleotid gebildet wird, das auf dem festen Träger immobilisiert ist, wodurch eine immobilisierte Zielpolynukleotid-cDNA gebildet wird;
(ii) Hybridisieren eines Erkennungsoligonukleotids an das immobilisierte Zielpolynukleotid, wodurch ein Erkennungsoligonukleotid-Zielpolynukleotid-Hybrid gebildet wird;
(iii) Spalten des Erkennungsoligonukleotid-Zielpolynukleotid-Hybrids mit einem Spaltmittel, das aus einem Restriktionsenzym ausgewählt ist, wodurch ein Gespaltenes-Erkennungsoligonukleotid-gespaltenes-Zielpolynukleotid-Hybrid gebildet wird, der ein gespaltenes Zielpolynukleotid beinhaltet;
(iv) Ligieren einer Adapternukleinsäuresequenz an das gespaltene Zielpolynukleotid, wodurch eine markierte cDNA-Sequenz gebildet wird,
wobei ein erstes Ankerpolynukleotid, das eine erste Amplifikationsnukleinsäuresequenz beinhaltet, kovalent an den festen Träger gebunden ist, und
wobei die Adapternukleinsäuresequenz ein erstes Amplifikationsnukleinsäuresequenzkomplement des ersten Ankerpolynukleotids beinhaltet; und
(v) Amplifizieren der cDNA-Sequenz durch klonale Amplifikation.

9. Verfahren gemäß einem der Ansprüche 1-8, wobei jeder Schritt in einer mikrofluidischen Vorrichtung durchgeführt wird.

10. Verfahren gemäß Anspruch 8, wobei das Zielpolynukleotid eine Vielzahl von Nukleinsäuresequenzen ist, wobei jedes der Zielpolynukleotide unabhängig unterschiedlich ist, wobei die Vielzahl von Zielpolynukleotiden von einer isolierten Zelle abgeleitet ist.

11. Verfahren gemäß einem der Ansprüche 1 bis 10, das ferner die folgenden Schritte beinhaltet:
- Amplifizieren der markierten Nukleinsäuresequenz durch Brückenamplifikation, beinhaltend das Hybridisieren des ersten Amplifikationsnukleinsäuresequenzkomplements an eine erste Amplifikationsnukleinsäuresequenz des ersten Ankerpolynukleotids; und
- Sequenzieren der markierten Nukleinsäuresequenz unter Verwendung von Sequenzierung durch Synthese.

12. Verfahren gemäß Anspruch 8, das ferner den Schritt des Sequenzierens des Amplifikationsprodukts von Schritt (v) beinhaltet.

## Revendications

1. Un procédé de formation d'une séquence d'acides nucléiques étiquetée, ledit procédé comprenant :
(i) l'immobilisation d'un polynucléotide cible sur un support solide, comprenant
(a) la capture d'une molécule d'ARN sur un support solide, formant de ce fait un ARN capturé,
(b) la transcription inverse dudit ARN capturé, puis le retrait de l'ARN capturé, formant de ce fait un polynucléotide cible immobilisé sur ledit support solide, formant de ce fait un ADNc de polynucléotide cible immobilisé ;
(ii) l'hybridation d'un oligonucléotide de reconnaissance audit polynucléotide cible immobilisé, formant de ce fait un hybride oligonucléotide de reconnaissance-polynucléotide cible ;
(iii) le clivage dudit hybride oligonucléotide de reconnaissance-polynucléotide cible avec un agent de clivage sélectionné parmi une enzyme de restriction, formant de ce fait un hybride oligonucléotide de reconnaissance clivé-polynucléotide cible clivé comprenant un polynucléotide cible clivé ; et
(iv) la ligature d'une séquence d'acides nucléiques d'adaptateur audit polynucléotide cible clivé, formant de ce fait une séquence d'acides nucléiques étiquetée ;
dans lequel un premier polynucléotide d'ancrage comprenant une première séquence d'acides nucléiques d'amplification est lié par covalence audit support solide, et
dans lequel ladite séquence d'acides nucléiques d'adaptateur comprend un premier complément de séquence d'acides nucléiques d'amplification dudit premier polynucléotide d'ancrage.

2. Le procédé de la revendication précédente, dans lequel ladite molécule d'ARN est extraite d'une cellule isolée.

3. Le procédé de n'importe quelle revendication précédente, dans lequel ledit support solide comprend une structure en bille, facultativement dans lequel ladite structure en bille est une bille de biotine.

4. Le procédé de n'importe quelle revendication précédente, dans lequel ledit premier polynucléotide d'ancrage comprenant une première séquence d'acides nucléiques d'amplification comprend en outre une première séquence d'acides nucléiques de libération, facultativement dans lequel ladite première séquence d'acides nucléiques de libération raccorde ladite première séquence d'acides nucléiques d'amplification audit support solide, facultativement dans lequel ladite première séquence d'acides nucléiques de libération comprend une séquence de clivage d'enzyme de restriction.

5. Le procédé de la revendication 1, comprenant l'hybridation dudit premier complément de séquence d'acides nucléiques d'amplification à ladite première séquence d'acides nucléiques d'amplification dans des conditions permettant une amplification PCR, amplifiant de ce fait ladite séquence d'acides nucléiques étiquetée.

6. Un procédé de la revendication 1 dans lequel la séquence d'acides nucléiques étiquetée est une pluralité de polynucléotides hétérogènes étiquetés, ledit procédé comprenant :
(i) l'immobilisation d'une pluralité de polynucléotides cibles hétérogènes sur un support solide tel qu'énoncé à l'étape (i) (a) et (b) de la revendication 1, formant de ce fait une pluralité de polynucléotides cibles hétérogènes immobilisés ;
(ii) l'hybridation d'une pluralité d'oligonucléotides de reconnaissance hétérogènes auxdits polynucléotides cibles hétérogènes immobilisés, formant de ce fait une pluralité d'hybrides oligonucléotide de reconnaissance-polynucléotide cible ;
(iii) le clivage desdits hybrides oligonucléotide de reconnaissance-polynucléotide cible avec un agent de clivage sélectionné parmi une enzyme de restriction, formant de ce fait une pluralité d'hybrides oligonucléotide de reconnaissance clivé-polynucléotide cible clivé ; et
(iv) la ligature d'une séquence d'acides nucléiques d'adaptateur à ladite pluralité de polynucléotides cibles clivés, formant de ce fait une pluralité de polynucléotides hétérogènes étiquetés,
dans lequel ledit acide nucléique d'adaptateur comprend au moins un premier complément de séquence d'acides nucléiques d'amplification d'un premier polynucléotide d'ancrage lié par covalence à un support solide.

7. Le procédé de la revendication 6, dans lequel ledit support solide comprend une structure en bille.

8. Un procédé d'amplification d'une séquence d'ADNc, ledit procédé comprenant :
(i) l'immobilisation d'un polynucléotide cible sur un support solide, comprenant
(a) la capture d'une molécule d'ARN sur un support solide, formant de ce fait un ARN capturé,
(b) la transcription inverse dudit ARN capturé, puis le retrait de l'ARN capturé, formant de ce fait un polynucléotide cible immobilisé sur ledit support solide, formant de ce fait un ADNc de polynucléotide cible immobilisé ;
(ii) l'hybridation d'un oligonucléotide de reconnaissance audit polynucléotide cible immobilisé, formant de ce fait un hybride oligonucléotide de reconnaissance-polynucléotide cible ;
(iii) le clivage dudit hybride oligonucléotide de reconnaissance-polynucléotide cible avec un agent de clivage sélectionné parmi une enzyme de restriction, formant de ce fait un hybride oligonucléotide de reconnaissance clivé-polynucléotide cible clivé comprenant un polynucléotide cible clivé ;
(iv) la ligature d'une séquence d'acides nucléiques d'adaptateur audit polynucléotide cible clivé, formant de ce fait une séquence d'ADNc étiquetée,
dans lequel un premier polynucléotide d'ancrage comprenant une première séquence d'acides nucléiques d'amplification est lié par covalence audit support solide, et
dans lequel ladite séquence d'acides nucléiques d'adaptateur comprend un premier complément de séquence d'acides nucléiques d'amplification du premier polynucléotide d'ancrage ; et
(v) l'amplification de la séquence d'ADNc par amplification clonale.

9. Le procédé de n'importe lesquelles des revendications 1 à 8, dans lequel chaque étape est effectuée dans un dispositif microfluidique.

10. Le procédé de la revendication 8, dans lequel ledit polynucléotide cible est une pluralité de séquences d'acides nucléiques, chacune étant de polynucléotides cibles qui sont différents indépendamment, la pluralité de polynucléotides cibles étant dérivée d'une cellule isolée.

11. Le procédé de n'importe laquelle des revendications 1 à 10, comprenant en outre l'étape
- d'amplification de la séquence d'acides nucléiques étiquetée par amplification en pont comprenant l'hybridation du premier complément de séquence d'acides nucléiques d'amplification à une première séquence d'acides nucléiques d'amplification du premier polynucléotide d'ancrage ; et
- de séquençage de la séquence d'acides nucléiques étiquetée à l'aide d'un séquençage par synthèse.

12. Le procédé de la revendication 8, comprenant en outre l'étape de séquençage du produit d'amplification de l'étape (v).
